# EUROPEAN PATENT SPECIFICATION

(11) **EP 3 294 354 B1**
(45) Date of publication and mention of the grant of the patent: **05.01.2022**
(21) Application number: 16724100.9
(22) Date of filing: 13.05.2016
(51) Int. Cl.: A61L 27/04, A61L 27/06, A61L 27/50, A61L 27/54, C25D 11/02, C25D 11/34

(54) **METAL OBJECT WITH ROUGHENED SURFACE AND METHOD OF PRODUCTION**
METALLOBJEKT MIT AUFGERAUTER OBERFLÄCHE UND VERFAHREN ZUR HERSTELLUNG
OBJET MÉTALLIQUE À SURFACE RUGOSIFIÉE ET PROCÉDÉ DE FABRICATION

(30) Priority: 15.05.2015 GB 201508385
(43) Date of publication of application: 21.03.2018
(73) Proprietor: Accentus Medical Limited, Didcot, Oxfordshire OX11 0DF (GB)
(72) Inventor: TURNER, Andrew Derek, Didcot Oxfordshire OX11 0DF (GB); MEROTRA, James, Didcot Oxfordshire OX11 0DF (GB); LEWIS, David Richard, Didcot Oxfordshire OX11 0DF (GB); SPEED, Jonathon, Didcot Oxfordshire OX11 0DF (GB); SHAWCROSS, James, Didcot Oxfordshire OX11 0DF (GB)
(74) Representative: J A Kemp LLP
(86) International application number: PCT/GB2016/051394
(87) International publication number: WO 2016/185186

(56) References cited:
- EP-A1- 2 495 356
- WO-A2-2012/095672
- US-A1- 2009 093 881
- US-A1- 2013 240 365

## Description

### FIELD OF THE INVENTION

The present invention relates to a method of treatment of a metal object to provide it with bio-effective properties, and to a metal object which incorporates a bio-effective material. This can provide a reduced risk of infection when the object is in contact with a body.

### BACKGROUND

Metal materials come into contact with the body in numerous situations. For example in surgery, where implants are used, these implants may be inserted into soft or hard tissue of the body. In the case of cancer treatment of the bone for example, cancerous bone tissue is removed, and a prosthetic metal implant is used to replace that part of the bone that has been removed. Implants are also used for partial or full replacement of bones in joints (e.g. hips) and also in other fields such as dentistry and maxillofacial surgery. Implants and medical devices may also be used in cases of amputation or trauma and such devices may be percutaneous or transcutaneous. Examples of such implants include: cardiac implants, including active implants such as pacemakers; cranioplasty implants; access ports; spine implants (e.g. rods, plates, screws, cages); neurostimulator devices; percutaneous and transcutaneous devices (e.g. ports); cochlear implants; acetabular cups; veterinary implants, and titanium horseshoes. Implants for the foregoing (and other) uses may be of titanium metal or titanium alloys. Titanium metal and titanium alloys are biocompatible, relatively strong and relatively light.

Further, metal comes into contact with the body in the case of jewellery. Irritation and infection can occur not only for jewellery that pierces the body but also for jewellery that sits next to the skin if the wearer has sensitive skin.

As can be seen, in both the medical and jewellery fields, the use of metal which comes into contact with body tissue runs the risk of introducing infection, or infection occurring.

WO 2010/112908 describes a way of treating a metal object so that it has biocidal properties. The object is anodised in two stages, firstly to passivate it with the formation of a surface layer, and secondly to form pits in the surface layer, the second stage of anodising being performed at a lower voltage than the first stage: for example the passivating voltage may rise to 100 V, and the second stage may use a voltage of 30 V. After anodising, the object is subjected briefly to a reverse voltage between -0.2 and -0.7 V. The metal object is then contacted with a solution containing biocidal metal ions, which become incorporated into the surface layer.

The process described in WO 2010/112908 enables a consistent level of biocidal material to be incorporated into metal objects of certain metal alloys. However, with some metal alloys, it has been found that the process requires a prolonged time to complete the anodising, or that the results are not sufficiently consistent, particularly with the more passive alloys. It would therefore be advantageous to provide a process applicable to such alloys, and which will ensure a consistent level of loading of biocidal material.

WO 2012/095672 describes a method of treating a metal object so as to incorporate a biocidal material in leachable form in the surface, the method comprising anodising the metal object to form an integral surface layer and to form pits through the integral surface layer, and contacting the anodised metal object with a solution containing a biocidal material so as to incorporate said biocidal material into the surface layer.

A further desirable property of surgical implants is the ability to promote integration into living tissue after implantation. This is particularly important in the case of implants which are intended to replace bone tissue, as bone implants must bear loads and so are vulnerable to damage unless they can undergo osseointegration and form a strong bond with the living bone. Similarly, implants intended for use in soft tissue which can integrate well with the surrounding soft tissue (for instance, the gingiva or soft gum tissue) are especially advantageous as they can facilitate wound healing and discourage the formation of scar tissue and thereby reduce the risk of infection.

It would therefore be advantageous to provide an implant which is capable of integration into living tissue, for example osseointegration, and which is also capable of providing a consistent and biologically acceptable (that is, safe and efficacious) level of biocidal material into the living tissue after implantation.

It is therefore desirable to provide an object having excellent biocidal and tissue integration properties. It is particularly desirable that the bio-effective material (such as a biocidal material) incorporated in such an object can easily elute into the local environment and prevent biofilm formation. It is further desirable to provide a method of producing such an object as quickly and easily as possible, and consistently across a wide range of materials.

### SUMMARY OF THE INVENTION

The present inventors have found that advantageous results may be obtained by introducing a voltage cycle to the surface treatment method. Specifically, it has been found that subjecting the passivated metal object to a cyclic voltage rather than a constant voltage can reduce the time taken to form the surface layer upon the object and achieve a more consistent process . The scope of this invention is defined by the claims. Thus according to the present invention there is provided a method of treating a metal object so as to form thereon a surface layer which is integral with the metal object, and which includes a bio-effective material, the method comprising the following steps:
(a) contacting the metal object having a micro-rough surface comprising microscale protrusions with an anodising electrolyte, and applying an anodising voltage to the metal object to passivate the metal by forming an anodised oxide layer on the metal object;
(b) continuing the application of an anodising voltage to produce regions in the oxide layer having a higher O/M ratio, where O is the number of oxygen atoms and M is the number of valve metal atoms;
(c) producing a hydrous metal oxide in said regions in the oxide layer by electrochemical or chemical reduction in contact with an electrolyte or a solution, so the oxide layer and the hydrous metal oxide in said regions together constitute the surface layer;
(d) removing or separating the anodised metal object resulting from step (c) from the electrolyte or the solution of step (c); and
(e) contacting the anodised metal object with a solution containing a bio-effective material so as to incorporate said bio-effective material into the surface layer;
*wherein* during step (b) the anodising voltage is repeatedly subjected to voltage cycles, each voltage cycle comprising ramping the voltage between a lower threshold voltage and an upper threshold voltage, and then returning the voltage to the lower threshold voltage, both the lower threshold voltage and the upper threshold voltage being less than the maximum voltage applied during the passivating step (a).

In the case of metal objects having surfaces which are polished, or do not have microscale roughness, the regions having a higher O/M ratio formed during step (b) take the form of pits through the oxide layer and into the substrate, and the hydrous metal oxide produced in step (c) is produced in said pits.

According to the claimed invention, the inventors have shown that the above technique which is applied to an object with a roughened surface produces surprisingly advantageous results. The rough surfaces which yield these advantageous results, referred to herein as "micro-rough" surfaces, are identified by the presence of microscale protrusions, in particular surfaces which have a high density of small protrusions. It was expected that application of the above-described method of the invention, or earlier methods as described for instance in WO 2009/044203, to such surfaces would result in the adsorption of a large amount of a bio-effective material such as silver upon the surface, for instance by adsorption of bio-effective material within the surface valleys and peaks. An object treated according to said method was therefore expected to be entirely unsuitable for use as an implant, due to excessive biocide loading and a loss of surface roughness. However, it was found that this technique in fact created a surface which maintained its rough structure and yet incorporated an amount of bio-effective material that was surprisingly lower and less toxic than expected.

Moreover, the inventors surprisingly found that, rather than forming pits upon this micro-rough surface, the method resulted in formation of regions in the oxide layer having a high oxygen to metal atom ratio (i.e. a high O/M ratio, wherein O is the number of oxygen atoms and M the number of valve metal atoms) upon the protruding portions of the surface; that is, the anodisation process led to more extensive anodisation at the peaks of the roughened surface and a greater oxygen atom content at these positions. O and M denote the number of oxygen and valve metal atoms respectively in the oxide layer at the region of interest. O and M may be measured, for example, by SEM-EDX. The presence of a higher oxygen to metal atom ratio at the protruding portions of the surface compared to the non-protruding portions of the surface may be described herein in terms of the formation of oxide caps. By contrast, in the case of a polished surface, or other types of surface which are not micro-rough as defined herein, regions having a particularly high oxygen to metal atom ratio occur where dips form through the oxide layer and into the metal surface. Such regions are described herein as pits. Treated metal objects having either pits or caps have been shown to effectively elute bio-effective materials (for example silver) into the surroundings, for example into surrounding tissue.

Incorporation of bio-effective material occurs in these regions of high oxide content (that is, high O/M ratio), being pits or caps. The incorporation of bio-effective material at the outermost parts of the surface layer, for example at protrusions upon the surface, is advantageous as it is likely to be more available to deter biofilm formation and to elute into the local environment.

Thus the invention also provides a metal object having a micro-rough surface and an anodised surface layer thereon which is integral with the metal object, wherein:
the rough surface of the metal object comprises microscale protrusions;
the integral surface layer comprises at least one oxide of the metal, and the oxygen in the surface layer is non-uniformly distributed across the surface of the metal object such that there are regions having a higher O/M ratio than the average O/M ratio in the anodised surface layer, where O is the number of oxygen atoms and M is the number of valve metal atoms, the regions being located on said microscale protrusions; and
a bio-effective material is incorporated into the regions having a higher O/M ratio.

The invention further provides a metal object of the invention obtainable according to the method of the invention.

### DESCRIPTION OF THE FIGURES

The invention will now be further and more particularly described, by way of example only, with reference to the accompanying figures, in which:
Figure 1 shows a graphical representation of the voltages used during surface treatment of a polished titanium alloy disc, showing also the cumulative charge passed;
Figure 2 shows a graphical representation of the current that flowed as a result of the applied voltages as shown in figure 1;
Figure 3 shows an EDX spectrum from the disc subjected to the treatment shown in figures 1 and 2;
Figure 4 shows a graphical representation of the voltages used during surface treatment of a grit-blasted titanium alloy disc, showing also the cumulative charge passed;
Figure 5 shows a graphical representation of the current that flowed as a result of the applied voltages as shown in figure 4;
Figure 6 shows a graphical representation of the variation of silver loading with the applied reverse voltage for a titanium/niobium alloy;
Figures 7a, 7c and 7e show SEM images (at magnifications of 1000x, 4000x and 15000x respectively) of a "GBA conditioned" titanium disc which was grit-blasted, acid-etched, and aged in NaCl solution for several months;
Figures 7b, 7d and 7f show SEM images (at magnifications of 1000x, 4000x and 15000x respectively) of a GBA conditioned titanium disc which was also anodised and silver-doped according to the method of the invention;
Figures 8a, 8c and 8e show SEM images (at magnifications of 1000x, 4000x and 15000x respectively) of a "GBA" titanium disc which was grit-blasted and acid-etched;
Figures 8b, 8d and 8f show SEM images (at magnifications of 1000x, 4000x and 15000x respectively) of a GBA titanium disc which was also anodised and silver-doped according to the method of the invention;
Figures 9a and 9c show SEM images (at magnifications of 1000x and 4000x respectively) of an "MF-CpTi" titanium disc which was machine-finished to create a smooth surface;
Figures 9b, 9d and 9e show SEM images (at magnifications of 1000x, 4000x and 15000x respectively) of an MF-CpTi titanium disc which was also anodised and silver-doped according to the method of the invention; and
Figure 10a shows the O/Ti atom ratio as measured by SEM-EDX at the peaks and troughs of a GBA conditioned titanium disc, after treatment according to the method of the present invention.
Figure 10b is as Figure 10a, except that the GBA conditioned titanium disc for which the data is shown is a blank (that is, not subject to treatment).
Figure 10c shows what is meant by troughs and peaks in the surface profile.
Figure 11a shows the silver content (in atomic %) as measured by SEM-EDX at the peaks and troughs of a GBA conditioned titanium disc, after treatment according to the method of the present invention.
Figure 11b is as Figure 11a, except that the GBA conditioned titanium disc for which the data is shown is a blank (that is, not subject to treatment).
Figure 12 contains two diagrams illustrating the location of the regions of higher oxygen to metal atom ratio (O/M ratio) on the surfaces of two metal objects according to the invention.

The upper diagram shows a rough metal object having an oxide cap (C) upon a protruding portion of the surface. The lower diagram shows a smooth metal object having wherein the region of high O/M ratio is located in a pit (P).

### DETAILED DESCRIPTION OF THE INVENTION

The method and metal object of the invention are described in more detail below.

### The method of the invention

The method of the invention involves the application of a first static voltage to a metal object in step (a), followed by a cyclically-varying voltage in step (b). Throughout the voltage cycles, the lower threshold voltage is preferably below 25 V, while the upper threshold voltage is preferably above 35 V. However, in an aqueous electrolyte oxygen evolution will occur at a voltage which depends upon the chemical constitution of the electrolyte, so the upper threshold voltage is preferably below the voltage at which oxygen evolution occurs. For example with an aqueous phosphoric acid electrolyte oxygen evolution occurs at about 80 V, for example between 80 V and 90 V. For example the upper threshold voltage may be between 30 V and 70 V, for instance between 35 V and 70 V or between 40 V and 70 V, for example 50 V or 60 V. The lower threshold voltage may be 0 V. So in one example the voltage is repeatedly ramped up from 0 V to 60 V, and then returned to zero again.

It has been found that this repeated ramping of the voltage gives consistent results with a wide range of different alloys, in particular more passive titanium alloys or pure titanium, in particular more passive titanium alloys, and that it provides a more rapid process than can be achieved by holding the anodising voltage at any fixed value. A potential explanation is that the formation of pits is a two-step reaction, and that the two steps occur at different voltages between 10 V and 50 V, possibly between 25 V and 35 V.

While ramping the voltage, the voltage is preferably varied continuously. It may be increased at a steady rate, or a rate which varies. By way of example the voltage may be varied at a rate between 0.1 and 10 V/s or 0.1 and 20 V/s, for example between 0.1 and 15 V/s, preferably between 0.5 and 5 V/s. Suitable ramp rates would therefore be 1, 2 or 3 V/s, although the process is more rapid the lower the ramp rate. The voltage may be decreased back to the lower threshold voltage more rapidly than it is increased during the ramping of the voltage. For example the voltage may be decreased five or ten times more rapidly than it is increased.

The number of voltage cycles to which the metal object is treated may be more than 10, and may be more than 20; in some cases as many as 100, 500, or more voltage cycles may be used. Consistent results may be obtained by monitoring the total electrical charge that has passed during the application of the anodising voltages in steps (a) and (b), and/or that during the reduction step (c), and ensuring that the total electrical charge per unit area lies within a desired range.

The reduction step, step (c), as a first option, comprises applying a negative voltage to the metal object that has been anodised during steps (a) and (b), while the metal object remains in contact with the anodising electrolyte. As a second option, the metal object that has been anodised during steps (a) and (b) may be put into contact with an electrolyte solution containing a reducible soluble salt of titanium or of the substrate metal, and subjected to a negative voltage to bring about electrochemical reduction. As a third option for step (c), instead of performing electrochemical reduction, the metal object may be contacted with a chemical reducing agent, after steps (a) and (b).

After anodisation in steps (a) and (b), it is believed that the surface layer regions (e.g. pits or caps, in particular pits) contain a peroxy cationic complex of the substrate metal. This complex can be reduced electrochemically to a hydrous metal oxide of limited solubility, as in the first option for step (c) described above. This complex can similarly be reduced chemically, as in the third option for step (c). Rather than relying on this complex remaining in the regions (e.g. pits or caps, in particular pits), in the second option for step (c) an electrolyte solution is provided that contains a peroxy cationic complex, preferably a peroxytitanyl, which can be reduced electrochemically in the regions (e.g. pits or caps, in particular pits) to hydrous titania.

Instead of using an external source of electricity for electrochemical reduction, the metal object may be made negative by connecting it electrically to an electrode of a corrodible metal, such as iron or steel; this may be immersed in the same electrolyte as the metal object, or in a separate electrolyte with ionic connection through a salt bridge or an ion-selective membrane. The corrodible metal electrode corrodes preferentially, so causing electrochemical reduction at the surface of the metal object.

In the case of chemical reduction (the third option for step (c)), where again a hydrous metal oxide is produced, the chemical reducing agent may be selected from one or more of the following: sodium sulphite, ferrous salts (chloride or sulphate), sodium nitrite, potassium bromide or iodide, or sodium borohydride or hydrazine. Stannous chlorides or sulphates, chromous chlorides or sulphates, or vanadous sulphates may be used under suitable conditions, although these have the disadvantage of polyvalent residues that may adsorb on the resulting hydrous titania.

Whichever option is selected for the reduction step, step (c), it results in the formation of hydrous metal oxides that have a high surface area. The high surface area provides for enhanced absorptive capacity for the bio-effective material. The bio-effective material may be one that has a biocidal effect, for example a metal ion that exhibits the oligodynamic effect. A suitable metal ion is silver. The high surface area material produced by the reduction step, step (c), allows for increased ion exchange with materials such as silver in ionic form. Under some circumstances, depending on the electrolyte used, the hydrous metal oxide may be combined with anions such as phosphate or sulphate, and this has similar ion exchange properties.

A bio-effective material that is biocidal, such as silver (Ag), can have a broad spectrum of activity. The silver ion is known for its biocidal effectiveness, especially at low concentrations (which may be referred to as the oligodynamic effect), killing pathogens or infectious agents. In some cases its effect may instead be that of controlling or inhibiting pathogens or infectious agents that cause infection. This means that the method of the present invention can produce a biocidal treated surface that can be effective at inhibiting the formation of biofilm on said surface, whilst also creating a local zone of inhibition as the silver elutes into the surrounding areas e.g. soft tissue. It is envisaged that other bio-effective materials may instead be provided, for example stem cells or proteins might be adsorbed on the hydrous titania surface that could also release nutrients into a site in the body, which may assist in infection control, healing or enhance biocompatibility where the implant is positioned in the body. The bio-effective material might instead have bacteriostatic properties, i.e. preventing the growth of bacteria, rather than killing bacteria.

The anodising is a two stage process with step (a) comprising the initial process of passivation i.e. growing a surface oxide layer, and then step (b) involving the formation of regions in the oxide layer having a high oxygen to metal atom ratio, for example the formation of pits formed through said oxide layer into the substrate metal, which corresponds to a reference embodiment, or in the case of surfaces having microscale protrusions involving the formation of caps on said protrusions, which is according to the claimed invention.

The maximum voltage applied during anodisation determines the thickness of the passive oxide layer. Lower voltages applied subsequently do not affect the thickness of the oxide layer. The maximum voltage may be as high as 2000 V, but is more typically between 80 V and 150 V, for example 100 V.

The voltage during passivation, step (a), may be applied as a voltage increasing linearly with time to a maximum, limiting value, or alternatively stepped voltages up to the maximum limit. It is also envisaged that multiple passivations may be used, where a voltage is applied repeatedly to prepare the metal surface for pitting, or for the formation of caps. These different ways of applying a voltage all come within the definition of applying an anodising voltage.

Preferably, in step (d), the metal object is rinsed to remove any electrolyte or solution remaining on the surface after the preceding steps. The rinsing may use water or any appropriate solvent. Then, in step (e), there is contact with the solution containing the bio-effective material. After step (e), the metal object may be rinsed again. The metal object, in the case of an implant, would at this stage be suitable for use, but it will be appreciated that it may then be subjected to further processing steps as commonly provided to medical devices, such as drying, packaging, further cleaning, and sterilisation; these further processing steps should be designed to not materially alter the chemical composition of the integral surface layer, and preferably would not alter the chemical composition of the integral surface layer.

The bio-effective material may be a biocidal material, for example a biocidal metal and in particular, the biocidal metal may be or may comprise silver, although other metals may be used in addition to or as alternatives to silver, for example a combination of copper and silver. Preferably, the biocidal material (e.g. metal, such as silver) is provided in the solution of step (e) in the form of ions.

As a result of the anodising and subsequent steps, the metal object has an integral surface layer formed of a hard anodised oxide layer, grown out from the surface, and regions therein having a higher oxygen to metal atom ratio (e.g. pits through this oxide layer or caps upon microscale protrusions, in particular pits) containing a hydrous metal oxide matrix that can absorb ions of the biocidal material, such as silver ions. The matrix contained within the regions of high oxygen to metal atom ratio (e.g. pits or caps, in particular pits) receiving the biocidal material may be soft and porous in comparison to the hard oxide surface, and with greater ion exchange capacity, so that the metal object combines the properties of enhanced silver ion storage capacity with the hard-wearing anodised surface. The anodised oxide layer, excluding the regions of high oxygen to metal atom ratio (e.g. pits or caps, in particular pits), can typically adsorb -0.3-1.0 µg/cm² Ag ions (on a microscopic area basis); the regions of high oxide content (e.g. pits or caps, in particular pits) make it possible to achieve a significantly higher loading, for example more than 5 µg/cm² Ag ions.

The magnitude of the negative voltage during electrochemical reduction, in step (c), is preferably maintained or regulated so as to be insufficient to cause electrolysis of the solvent. The magnitude of the negative voltage affects the absorptive capacity of the surface, as it affects the magnitude of the reducing current; the electric charge passed is directly related to the creation of the hydrous metal oxide absorber matrix, and hence to the amount of biocidal material (e.g. metal, such as silver) which can subsequently be incorporated. With some alloys it has been found that a somewhat larger negative voltage is desirable. This may be due to semiconductor properties of the surface oxide.

In particular, when treating an object formed of a titanium/niobium alloy it has been found that the optimum absorptive capacity is attained by applying a reverse voltage of about -0.8 V, whereas with pure titanium or a titanium/vanadium alloy, in particular a titanium/vanadium alloy, a voltage of -0.45 V is sufficient, when using 2.1 M aqueous phosphoric acid as the electrolyte.

Typically, the current drops during the application of the negative voltage, and the voltage is preferably applied until this current has fallen significantly, for example to less than 20% of the peak current. Typically this may take only 3 minutes or less. Indeed the current may drop to substantially zero.

The metal of the metal object may comprise titanium or may comprise another valve metal such as niobium, tantalum or zirconium, or an alloy comprising such a metal. The metal of the metal object may comprise other metals, for example the object might be of stainless steel plated or coated with a metal such as titanium or the other valve metals or their alloys. The invention also has application to metal articles that have already been anodised for example by Type I, Type II or Type III anodising. The metal of the metal object may be in the form of porous structures such as those manufactured using additive layer techniques including laser or electron-beam sintering. The invention is also applicable to fibres, hybrid metal structures comprising more than one type of metal, sintered bead structures, and other porous forms of titanium or other valve metals such as those created by plasma spraying. The method of the invention may in particular be applied to a metal implant manufactured by any means. Examples of implants include orthopaedic implants (typically cranioplastic implants or spine implants, e.g. rods, plates, screws or cages), dental implants; cardiac implants, including active implants such as pacemakers; access ports; neurostimulator devices; percutaneous and transcutaneous devices (e.g. ports); cochlear implants; acetabular cups; veterinary implants, and titanium horseshoes.

The metal object may have a machined finish. Techniques such as grit blasting or shot blasting or shot peening may also be used to prepare the surface (e.g. for subsequent application of hydroxyapatite by plasma spraying after biocidal ion loading, to stimulate localised bone attachment). According to the claimed invention, the surface is roughened before treatment in accordance with the invention.

According to the claimed invention, where the surface preparation technique leads to a micro-rough surface, for instance microscale protrusions, then the method of the invention leads to the formation of caps. Acid etching is an example of a technique which provides such a surface micromorphology. The results observed on applying the method of the invention to surfaces prepared by various methods are summarised in Table 1 below.

**Table 1. A summary of the effects seen upon applying the method of the invention to a range of surface types. In brief, the smoother surfaces (not according to the claimed invention) tended to produce pits while the acid-etched surface having microscale protrusions (according to the claimed invention) produced caps.**

| Surface Type | Polished | Machine-finished | Grit blasted | Acid etched |
|---|---|---|---|---|
| Result | Pits | Pits | Pits | Caps |

The anodising electrolyte preferably comprises phosphoric acid as a dilute solution of a desired pH in a solvent. The solvent may comprise water. Other electrolytes such as sulphuric acid, phosphate salt solutions or acetic acid may be used. Alkaline electrolytes such as sodium hydroxide may be used also. It is preferred that these electrolytes are in a diluted form for example 2.1 M H₃PO₄, or 0.1 M H₂SO₄.

The phosphoric acid may have a concentration in a range of from 0.01 M to 5.0 M, typically from 0.1 M to 3.0 M and in particular around 2 M (an alternative electrolyte is 0.01 to 0.3 M sulphuric acid). Preferably, the pH of the acidic electrolyte should be maintained within the range of 0.5 < pH < 2.0 - more ideally within the range 0.75 < pH < 1.75.

If an alkaline electrolyte is used the pH is preferably greater than 9 and more typically the pH is in the range of 10-14. The alkaline electrolyte can be a phosphate salt such as Na₃PO₄, or may be sodium hydroxide, e.g. 0.1 M NaOH

In instances where other metal substrates or anodising electrolytes are used instead of phosphoric acid, sulphuric acid or acetic acid, the magnitude of the negative (i.e. reverse) voltage may need to be adjusted to provide the desired effects due to factors such as changes in pH, or even temperature.

The geometric surface area of the metal object can be determined by conventional means such as the use of standard measuring devices such as callipers, micrometers and rulers combined with a geometric model of the item being treated e.g. using Computer Aided Design (CAD), or more advanced optical methods such as laser scanning. In some contexts, for example when describing an implant, the silver loading per geometric area may be an appropriate parameter. This measurement does not however take into account microscopic surface features or surface roughness of the metal.

The ratio of microscopic to geometric area is known as the surface roughness factor (RF). This microscopic surface area is an important factor in determining and controlling how much charge should be passed during the anodisation step. The microscopic surface area can be determined, for example, by immersion of the metal object in an electrolyte, applying a gradually increasing voltage to a low voltage such as 2.5 V to form a consistent and thin oxide layer, and deducing the microscopic surface area from electrical measurements. The surface area may be deduced from the film growth current; or subsequently may be deduced by measuring the double layer capacitance and comparing this to calibrated standards under identical conditions of temperature and electrolyte concentration. (Such a process for measuring the microscopic surface area is described in WO 2012/095672.) The charge or current per microscopic surface area e.g.Coulomb/cm² or mA/cm² is therefore typically used in the control of the anodising process. Hence the number of voltage cycles is preferably arranged to ensure a predetermined charge per unit area, based on the microscopic surface area. For different applications, the appropriate loading of bio-effective material, and so the degree of anodising, may be different. By way of example, to be sure that an implant has been sufficiently anodised, and so absorbs a sufficient level of silver ions, an anodising charge of between 0.1 and 10 C/cm², for example between 0.3 and 5 C/cm² should be passed, this being calculated on the basis of the microscopic surface area; this takes into account the charge passed in both steps (a) and (b). For example, a suitable anodising charge may be between 2.6 and 2.8 C/cm², for a polished surface; but for a rough, grit-blasted surface a suitable anodising charge may be between 0.4 and 0.6 C/cm² (on a microscopic basis).

During the passivation step (a), the current may be controlled, or alternatively the voltage may be controlled. The anodising may be performed with a limiting microscopic current density in a range of from 0.1 to 100 mA/cm², preferably 0.1 to 50 mA/cm², or more typically 1 to 10 mA/cm², e.g. 5 mA/cm² or thereabouts. Alternatively, an applied voltage linearly increasing with time, preferably at between 0.1 V/s and 10 V/s, for example at 1 V/s or 0.5 V/s, or increasing in steps, may be applied to control the passivation process. In either case the applied potential is raised to a maximum value (e.g. of 100 V). When the desired maximum voltage has been reached, the voltage is held constant, and as the passivation reaches completion the current falls to a significantly lower value. The passivation step (a) may be considered to be complete when the current has decreased to a low value.

### Application of the method of the invention to a micro-rough object

According to the present invention, the method of the invention is applied to a metal object having a micro-rough surface, i.e. having microscale protrusions. Microscale protrusions are protrusions which extend substantially perpendicularly to the surface plane. By "surface plane" is meant the mean surface plane. The mean surface plane is the average level of the surface. Protrusions may be in the form of peaks, which may be elongated or adjoined to form protruding ridges. The protruding ridges may be for example between 0.1 and 100 micrometres long. The micromorphology of the surface may be overlaid on a larger-scale structure, and may have nanostructures overlaid thereupon. Preferably these microscale features reach a fine point at their tip and thus have sharp edges when viewed on the microscale.

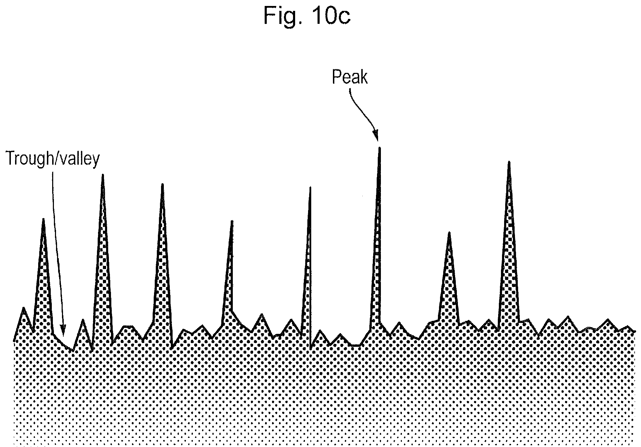

The micro-rough surface comprises troughs or valleys between the microscale protrusions. As used herein, the terms "trough" and "valley" indicate a region of the surface whose level is below the mean surface plane. Peaks and troughs are illustrated in Figure 10c.

The method of the invention leads to the formation of regions in the oxide layer having a higher oxygen to metal atom ratio on the microscale protrusions of a micro-rough surface. By "on the microscale protrusions" is meant that the region of higher O/M ratio is located at the portions of the microscale surface features which extend beyond the mean surface plane away from the surface. The regions of the surface layer present on the microscale protrusions may comprise various different oxide species including hydrous oxide compounds.

The O/M ratio is the ratio of the number of oxygen atoms to the number of valve metal atoms. A valve metal is a metal which is readily passivated with oxygen. A valve metal may typically be selected from titanium, zirconium, niobium or tantalum. The metal object of the invention typically comprises at least one valve metal, or at least one alloy comprising a valve metal.

The roughness of a surface may be measured in a variety of ways, including (i) contact methods using a stylus or (ii) non-contact methods, including focus detection, confocal microscopy or interferometry. It is also possible to observe the average roughness using scanning electron microscopy (SEM). These techniques yield a variety of parameters which quantify the roughness of the surface, and which are discussed below.

The micro-rough surface can be described in terms of its average roughness, or the arithmetic mean surface roughness (R_{A}), which is defined as the arithmetic average of the absolute values of the profile height deviations from the mean surface plane. R_{A} is typically measured by stylus, interferometry or SEM, preferably by interferometry. The metal object having a micro-rough surface typically has an average roughness R_{A} of from 0.5 to 10 µm, preferably from 0.5 to 5 µm, for example from 1 to 3 µm. R_{A} is typically measured by taking the average of profile height deviations measured at intervals across a linear slice across the surface; the equivalent average taken over an area is known as S_{A}, which is also typically measured by interferometry or SEM, preferably by interferometry.

A similar measure of surface roughness is given by the profile average (P_{A}) which is the arithmetic average of the raw measured profile. P_{A} is typically measured by interferometry or SEM, preferably by interferometry. Thus both P_{A} and S_{A} of the micro-rough surface may be for example from 0.5 to 10 µm, preferably from 0.5 to 5 µm, for example from 1 to 3 µm.

The micro-rough surface can alternatively be described in terms of the parameter S_{ds}, or density of summits. S_{ds} is defined in the Commission of the European Communities publication, "The development of methods for the characterisation of roughness in three dimensions", Stout *et al.,* Publication no. EUR15178-EN. S_{ds} is typically measured by interferometry or SEM, preferably by interferometry. This gives the number of peaks per square millimetre at the surface. The metal object having a micro-rough surface typically has a density of summits of at least 1000, for example from 5,000 to 1,000,000 mm⁻², preferably from 10,000 to 500,000 mm⁻², more preferably from 15,000 to 500,000 mm⁻² or from 20,000 to 500,000 mm⁻². Preferably the density of summits is at least 15,000 or at least 25,000 mm⁻², e.g. at least 50,000 or at least 100,000 mm⁻².

The micro-rough surface can alternatively be described in terms of the parameter Sₛₖ, or skewness of the height distribution, which is typically measured by interferometry or SEM, preferably by interferometry. A positive value of the skewness of the height distribution indicates that the surface contains distinct protrusions such as peaks, whereas a negative value indicates that the surface contains dips or valleys. The micro-rough surface preferably has a positive Sₛₖ value.

The micro-rough surface of the metal object may alternatively be described in terms of its microscopic area. The rougher the surface, the larger its microscopic area will be compared to its geometric surface area. The geometric surface area of a metal object can be determined by conventional means such as the use of standard measuring devices such as Computer Aided Design (CAD), callipers, micrometres and rulers combined with a geometric model of the item being treated, or more advanced optical methods such as laser scanning. The microscopic surface area can be determined, for example, by immersion of the metal object in an electrolyte, and measuring the double layer capacitance and comparing this to calibrated standards under identical conditions of temperature and electrolyte concentration. The ratio of microscopic to geometric area is known as the surface roughness factor (RF) and can be used to convert one area to the other. The metal object having a micro-rough surface typically has a surface roughness factor greater than 1.5, for example from 2 to 10.

Another method which can be used to determine the surface roughness factor is known as the pre-anodisation technique. The pre-anodisation technique involves the determination of the surface roughness factor using electrical measurements made while or after the surface of the metal object is contacted with an anodising electrolyte and pre-anodised to grow a thin oxide film. In more detail, the object whose surface area is to be determined is immersed in an electrolyte and subjected to a small, linearly increasing voltage while the current is measured. The current is monitored during this procedure and the surface area may be deduced therefrom. This technique is described in detail in WO 2012/095672 A2.

Where the surface roughness factor for a micro-rough surface is measured by the pre-anodisation technique, the surface roughness factor is typically greater than 1.5, for example from 2 to 10, preferably from 3 to 6.

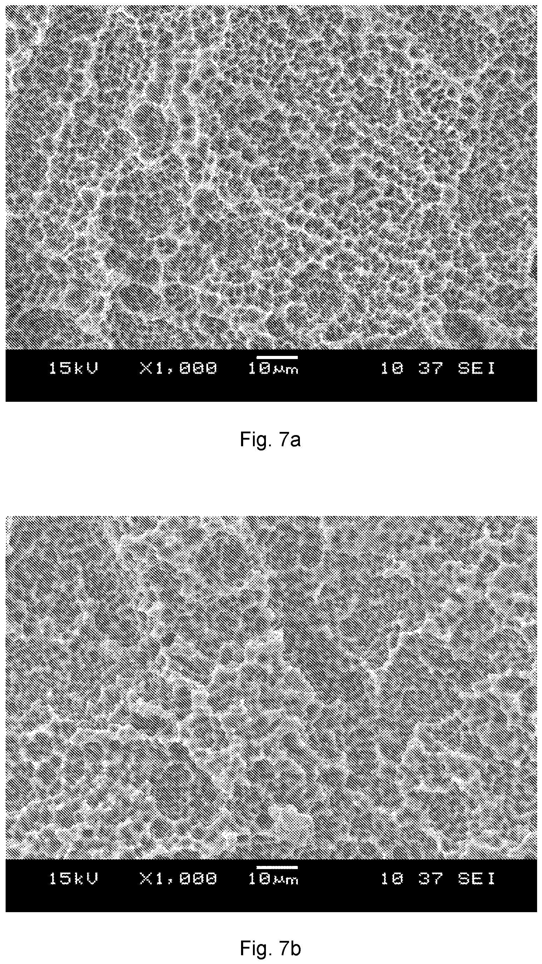

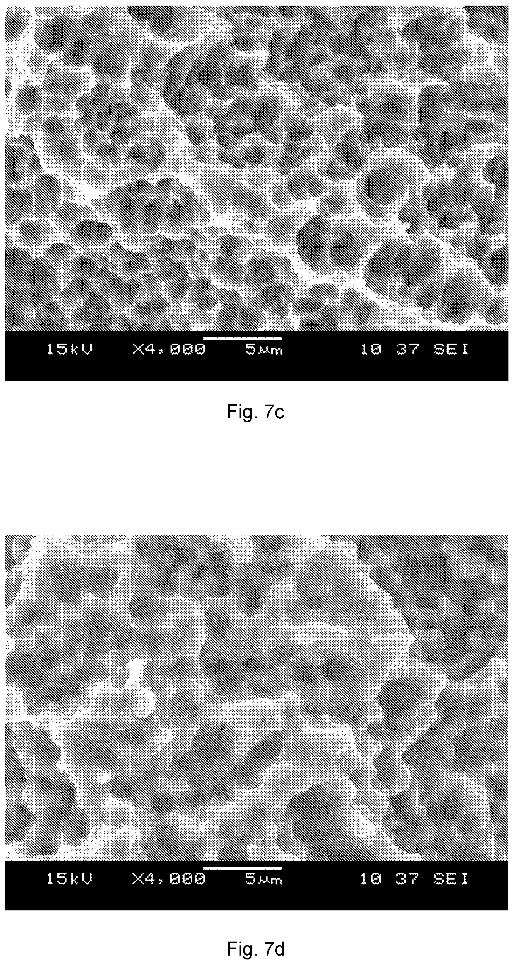

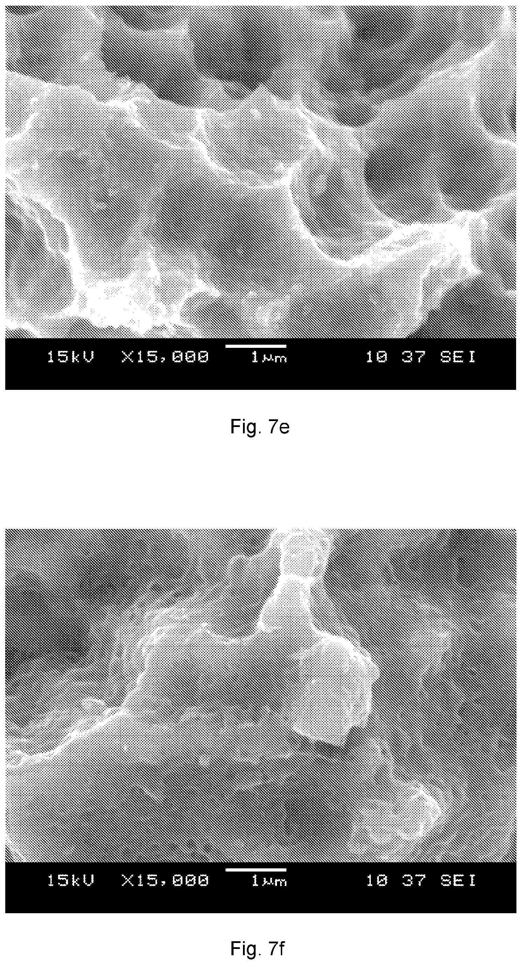

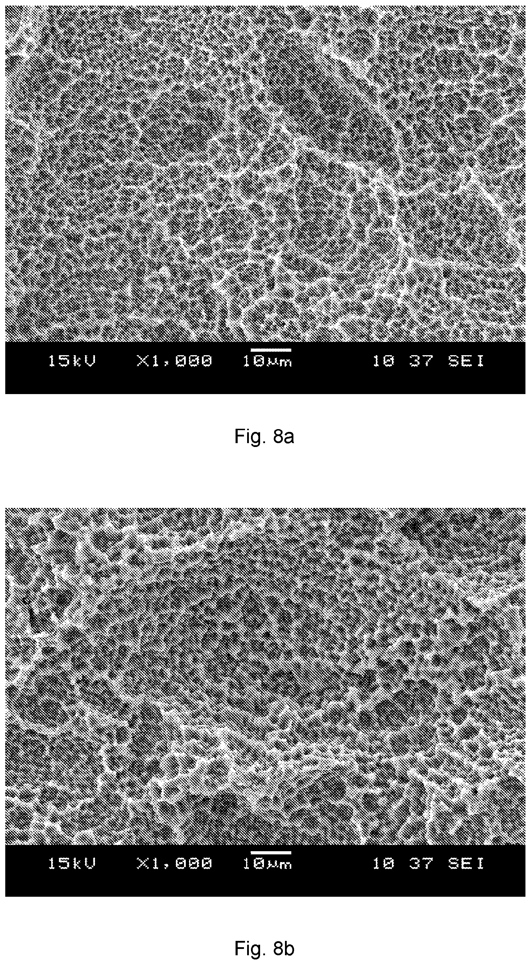

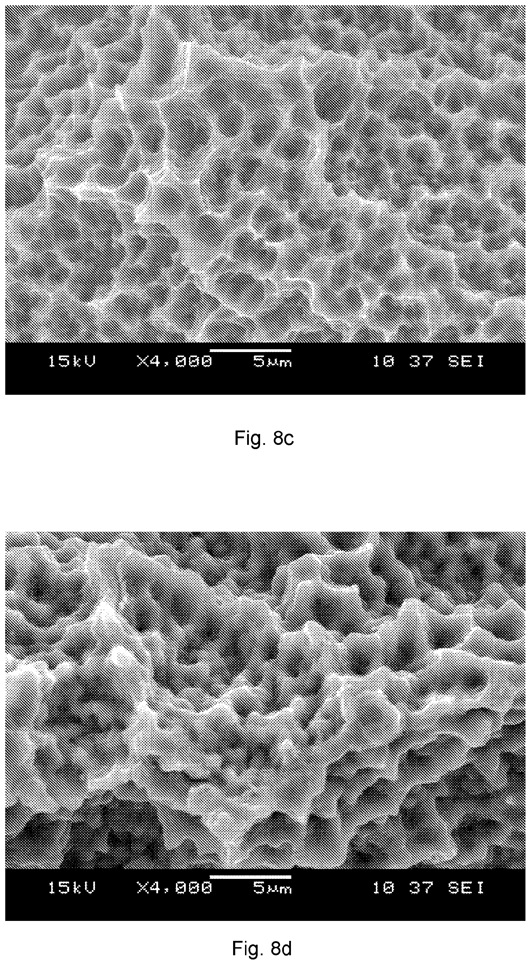

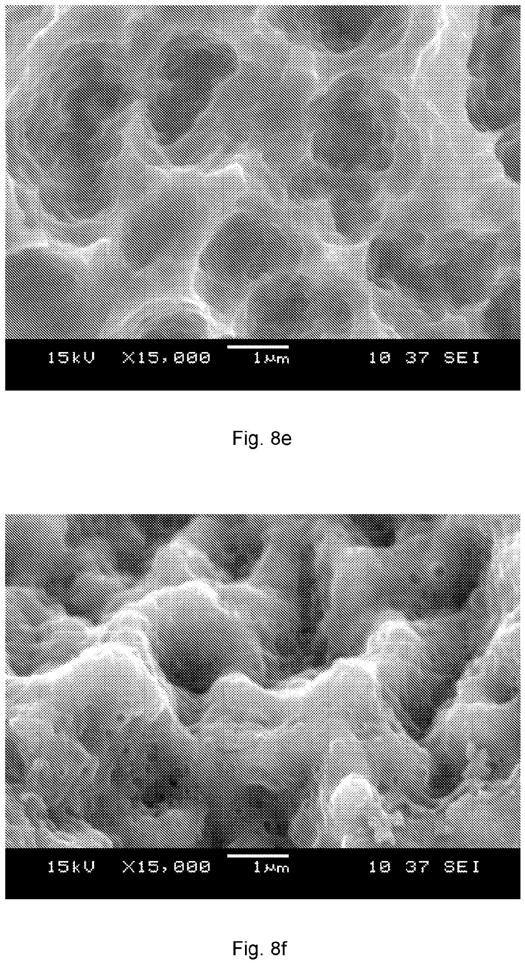

The pre-anodisation technique measures the surface area of the metal object prior to the creation of an integral surface layer by anodisation. It does not, therefore, directly measure the surface area of a metal object according to the invention. However, there is no significant difference between these surface areas. For example, in the case of polished surfaces, there is no statistical difference between the surface area of the metal object prior to treatment and the surface area of the metal object after treatment according to the method of the invention. It is further evident from the SEM images in Figures 7 and 8 that images taken before and after application of the method of the invention to a roughened metal surface having microscale protrusions that the protrusions simply become slightly rounded. The number of protrusions is unchanged by the method of the invention, and the slight rounding of the peaks does not significantly affect the surface area.

The ratio of the microscopic surface area to the geometric surface area is known as the developed surface area, S_{dr}, which is expressed as a percentage enlargement compared to the geometric surface area. The developed surface area is typically measured by interferometry or SEM, preferably by interferometry. The S_{dr} of the micro-rough surface is, for example, more than 50%, preferably from 80% to 300%, more preferably from 100% to 200%.

The following Table (Table 2) summarises typical ranges of values of the foregoing parameters in relation to surfaces prepared by various techniques.

**Table 2**

| **Parameter** | **Meaning** | **Polished** | **Grit Blasted** | **Plasma Sprayed** | **Grit blasted & Acid etched** |
|---|---|---|---|---|---|
| R_{A} (µm) | Arithmetic mean of deviations | 0.01 - 0.5 | 3.5 - 8.0 | 5 - 50 | 1.5 - 2.0 |
| RF (S_{dr}/100 +1) | Ratio of microscopic to geometric area | 1.1 - 1.5 | 4-10 | 50 - 100 | 3-10 |
| S_{DS} (mm⁻²) | Peak density | ~0-500 | 2000 - 10,000 | ∼10-250 | 25,000 - 300,000 |

The oxide caps of the metal objects of the invention are seen on micro-rough surfaces. Micro-rough surfaces are surfaces preferably having small, micron-sized protrusions, particularly surfaces having an R_{A} of from 0.5 to 5 µm, for example an R_{A} of up to 3 µm, e.g. from 1 to 3 µm. More preferably, micro-rough surfaces also have a high density of protrusions, particularly an S_{DS} of at least 1,000 mm⁻², e.g. at least ,5000 mm⁻², preferably at least 10,000 mm⁻², more preferably at least 15,000 or 20,000 mm⁻².

The micro-rough surface as defined herein has an S_{DS} of at least 1000 mm⁻², typically at least 5,000, 10,000, 15,000 or 20,000 mm⁻²; an R_{A} of 3 µm or less, e.g. from 1 to 3 µm; and an RF of 10 or less.

The microscale protrusions which appear at the surface of the micro-rough metal object may occur substantially uniformly over the surface of the object, or they may be present only in one or more portions of the object's surface. Where only one or more portions of the surface are roughened, the microscale protrusions may occur substantially uniformly across each portion.

The micromorphology of the metal object having a roughened surface may be produced by, for example, acid etching, treatment with a fluoride solution, lithography or laser pitting, preferably acid etching. In one embodiment, the micromorphology is obtained by a method other than plasma spraying. Alternatively, the metal object may be directly produced with a micro-rough surface, rather than by subsequent roughening of the surface of the object.

A variety of acids may be used in acid etching of a surface in order to roughen it. For example, hydrofluoric acid, hydrochloric acid, sulphuric acid or nitric acid, or any combination of these, may be used in acid etching. The acids preferred for use in acid etching are hydrochloric acid or sulphuric acid, or a mixture thereof. The concentration of the acid may vary up to 100%. For example, the concentration of acid may be at least 10%, 20% or 50%. Preferably, acid etching involves the use of acid in a boiling state.

The metal object may be subject to a combination of two or more roughening steps, one of which is typically an acid etching step. Typically, where multiple roughening steps are employed, the acid etching step is the final roughening step. For example, the metal object may be subject to grit blasting, sanding, or sand blasting followed by acid etching. A further advantage of an acid etching step in this case is that the acid etching step will remove any debris or grit left on the surface by the sand blasting step.

Thus in one embodiment, the method of the invention comprises an acid etching step prior to anodisation of the surface according to step (a). Preferably, the metal object is cleaned (such as in nitric acid) after the acid etching step and prior to step (a). More preferably, the metal object is cleaned (such as in nitric acid) and rinsed in deionised water after the acid etching step and prior to step (a).

If desired, the roughened surface may be conditioned after roughening, e.g. for up to several months in a solution of sodium chloride. Typically, an aqueous solution of sodium chloride or even just water is used. Typically, the conditioning process lasts up to 4 months, e.g. 2 weeks to 3 months, eg 1 to 3 months.

The surface may alternatively or additionally be subjected to various other treatment options including any one or more of lithography, laser pitting, or any combination of the foregoing. Thus in one embodiment, the surface is subject to any one of lithography or laser pitting prior to a roughening step, typically an acid etching step, whereas in another embodiment the surface is roughened by a roughening method (for example acid etching) prior to treatment by lithography and/or laser pitting.

In a further embodiment of the method of the invention, lithography may be utilised during a roughening step to enable only a portion of the metal object to be roughened prior to treatment according to the method of the invention.

When the metal object has a micro-rough surface comprising microscale protrusions, as required for the present invention, the anodising and subsequent steps create an integral surface layer which has a non-uniform composition across the surface of the metal object such that there are regions having a higher oxygen to metal atom ratio, or "caps", located upon the microscale protrusions. That is, in the course of steps (a) to (c), the anodisation process which forms the integral surface oxide layer results in a higher oxygen to valve metal atom ratio at the protrusions than at the parts of the surface which do not extend out of the surface plane. The treated metal object having said integral surface layer is described further below.

The bio-effective material is adsorbed into the integral surface layer in step (d). Where the method of the invention is applied to a micro-rough surface, the bio-effective material is adsorbed into the regions having a high oxygen to metal atom ratio or caps at the protrusions upon the surface. That is, the bio-effective material is adsorbed to a greater extent at the regions having a high oxygen to valve metal atom ratio than elsewhere upon the surface. The location of the bio-effective material may be observed, for example, by SEM-EDX.

### Treated metal objects

According to a further aspect of the invention, there is provided a metal object obtainable or obtained by the methods described above and hereinafter.

The metal object may be in the form of an implant, a medical implement or device or an item of jewellery. In particular, in the case of a medical implement or device, this could include any type of device or tool that comes into contact with the body, for example pace-makers, stents, skin staples, scalpels, trocars, pins for bones or even medical implements such as scalpels or tissue clamps which are used during surgery. Implants according to the invention can be used for many medical and surgical purposes, including full and partial hip replacements, implants useful in maxillofacial, trauma, urology, orthodontal and orthopaedic applications, arthroscopic devices, dental implants, neurological apparatus and parts (such as staples, nails and pins) used in cardiovascular and general surgery.

The metals that may be used to make the implants or jewellery according to the invention may be titanium, typically chemically pure (also referred to as commercially pure) titanium, for example, Grade 2 (ASTM F67), or a titanium alloy. One standard alloy for this purpose is titanium 90% with 6% aluminium and 4% vanadium (British Standard 7252). An alternative is titanium 87% with 6% aluminium and 7% niobium (ASTM 1295-11 or ISO 5832-11). Alternatively the metal may comprise niobium, tantalum or zirconium, or alloys of these metals. The percentages given are percentages by weight.

### Treated metal objects having a micro-rough surface

According to the invention, there is provided a metal object having a micro-rough surface and a surface layer thereon which is integral with the surface of the metal object, wherein the micro-rough surface of the metal object comprises microscale protrusions; the integral surface layer comprises at least one oxide of the metal and the oxygen in the surface layer is non-uniformly distributed across the surface of the metal object such that there are regions having a higher oxygen to metal atom ratio, the regions being located on the microscale protrusions; and a bio-effective material is incorporated into the integral surface layer.

The treated metal object according to this aspect of the invention may be in any of the forms discussed above, that is, an implant, a medical implement or device or an item of jewellery. For example, this could include any type of device or tool that comes into contact with the body such as pace-makers, stents, skin staples, scalpels, trocars, pins for bones or even medical implements such as scalpels or tissue clamps which are used during surgery. However, the micro-rough surface of the treated metal object makes it particularly suited to applications where integration with living tissue is required, such as osseointegration. Thus implants according to the invention can be used for many medical and surgical purposes, including full and partial hip replacements, implants useful in maxillofacial, trauma, urology, orthodontal and orthopaedic applications, arthroscopic devices, dental implants, neurological apparatus and parts (such as staples, nails and pins) used in cardiovascular and general surgery. Examples of preferred implants include: orthopaedic implants, typically spine implants (e.g. rods, plates, screws and cages) or cranioplasty implants; access ports; neurostimulator devices; percutaneous and transcutaneous devices; ports; cochlear implants; acetabular cups; veterinary implants; titanium horseshoes; dental implants; implants for soft tissue integration, and prosthetics such as intraosseous transcutaneous amputation prosthesis. Dental implants, cardiac implants and cranioplastic implants are particularly preferred.

Preferably, implants according to the invention or implants made according to the method of the invention are for insertion into the human body.

There is no particular limit on the shape of the metal object. Thus, it may be a hollow or solid object, or it may mimic the structure of bone, for example cancellous bone.

The metal object may comprise titanium, tantalum, zirconium, niobium, vanadium, aluminium, or alloys thereof. Among these, titanium and titanium alloys are particularly favoured. Chemically pure titanium is an example of a suitable metal, as are the alloys mentioned above (90% titanium, 6% aluminium and 4% vanadium or 87% titanium, 6% aluminium and 7% niobium). The percentages given are percentages by weight.

The treated metal object having a micro-rough surface with microscale protrusions has a micromorphology as described above in relation to the micro-rough metal object for use in the method of the invention. Thus the treated micro-rough surface typically has microscale protrusions which extend substantially perpendicularly to the surface plane. Such protrusions may be in the form of peaks, which may be elongated or adjoined to form protruding ridges. The protruding ridges may be for example between 0.1 and 100 micrometres long. The micromorphology of the surface may be overlaid on a larger-scale structure, and may have nanostructures overlaid thereupon. Preferably the microscale features have edges which appear sharp on the microscale.

As has been mentioned above, the application of the method of the invention to the metal object may cause a rounding of the peaks of the microscale protrusions, but does not substantially affect their size and has no effect on the number or density of the protrusions. In consequence, application of the method of the invention to an object having a micro-rough surface has a minimal effect on the surface roughness factor. For example, the method of the invention does not reduce the surface roughness factor by more than 10%. In particular, the method of the invention does not reduce the surface roughness of the metal object by more than 5%. Therefore the treated metal object having a micro-rough surface as described above, having a surface layer obtainable by the method of the invention, has a roughened surface which is substantially similar to the surface of the untreated metal object.

In particular, the treated micro-rough metal object typically has an average roughness (R_{A}) of from 0.5 to 10 µm, preferably from 0.5 to 5 µm, for example from 1 to 3 µm. The metal object typically has a density of summits of at least 1000 mm⁻², for example from 5,000 to 1,000,000 mm⁻², preferably from 10,000 to 500,000 mm⁻², more preferably from 15,000 to 500,000 mm⁻² or from 20,000 to 500,000 mm⁻². Preferably the density of summits is at least 15,000 or at least 25,000 mm⁻², e.g. at least 50,000 or at least 100,000 mm⁻². The treated micro-rough metal object preferably has a positive Sₛₖ value.

The treated metal object having a micro-rough surface typically has a surface roughness factor greater than 1.5, for example from 2 to 10. Where the surface roughness factor is measured by the pre-anodisation technique discussed above, it is preferably from 3 to 6. The treated micro-rough metal object preferably has a developed surface area (S_{dr}) measured by interferometry of, for example, more than 50%, preferably from 80% to 300 %, more preferably from 100% to 200%.

The microscale protrusions which appear at the surface of the treated micro-rough metal object may occur substantially uniformly over the surface of the treated object, or they may be present only in one or more portions of the treated object's surface. Where only one or more portions of the surface are roughened, the microscale protrusions may occur substantially uniformly across each portion.

The micromorphology of the micro-rough metal object is obtainable by, for example, acid etching, treatment with a fluoride solution, laser pitting or lithography, preferably by acid etching. This micromorphology is also obtainable by, for instance, a method comprising acid etching, for example a method comprising acid etching and at least one other roughening method as described above.

The treated metal object comprises an integral surface layer which comprises at least one oxide of the metal. An integral surface layer is a layer which is a part of the metal object, (rather than a separate coating applied to the metal object). Typically, the integral surface layer is an anodised layer. Further, the oxygen atoms in the surface layer are not uniformly distributed and so the surface layer comprises particular regions where the ratio of oxygen to valve metal atoms is higher than elsewhere on the surface, e.g. higher than the average oxygen to metal atom ratio in the integral surface layer. These regions are located at the peaks of the microscale protrusions such that there is a greater O/M ratio at the peaks. Thus, the surface structure comprises microscale protrusions at which the oxygen to metal atom ratio is high (and a high degree of oxidation driven by anodisation has taken place), and valleys between the protrusions where the oxygen to metal atom ratio is lower (and a lower degree of oxidation driven by anodisation has taken place). Typically, the regions of the integral surface layer which have an elevated oxygen to metal atom ratio in comparison to the average oxygen to metal atom ratio in the surface are for the most part distributed over the portions of the surface which protrude from the mean surface plane away from the surface. The occurrence of such regions is reduced, or they are even substantially not present, at those portions of the surface which are recessed into the surface below the mean surface plane.

The presence of the integral surface layer at the summits of the microscale surface protrusions means that the peaks and ridges of the surface have a rounded shape compared to those of a micro-rough metal surface without an integral surface layer. The rounded surface protrusions are visible when magnified. This may be seen in Figures 7 and 8, wherein the protrusions at the surface of the untreated GBA conditioned and GBA discs have sharp points and narrow ridges, whereas those features appear to have been smoothed over in images of the treated discs at the same magnification. The apices of the ridges are broader and the peaks appear blunted. This is due to the integral surface layer comprising oxide thereupon. Thus the micro-rough treated metal object of the invention comprises a surface having microscale features such as protrusions which are rounded by the presence of caps, being regions of higher oxygen to metal atom ratio than the rest of the surface.

Due to the uneven distribution of oxygen throughout the integral surface layer (typically the anodised layer), the ratio of oxygen atoms to valve metal atoms is greater at the surface protrusions than at the non-protruding portions of the roughened surface. Typically, the ratio of oxygen atoms to valve metal atoms is at least 1 in the surface layer of the metal object of the invention. The ratio of oxygen to valve metal atoms at the protrusions is typically at least 1.5, for example at least 2 or 2.5. The ratio of oxygen to valve metal atoms in the troughs between the peaks is typically from 0.5 to 2, for example from 1 to 1.5 or less than 1.5. Preferably, the ratio of oxygen to valve metal atoms is at least 2 at the protrusions and at least 1 at the troughs or non-protruding portions of the surface. More preferably, the ratio of oxygen to valve metal atoms is at least 2 at the protrusions and at least one, but less than 2, at the troughs or non-protruding portions of the rough surface.

The ratio of oxygen atoms to valve metal atoms is typically larger at the surface protrusions than at the non-protruding portions of the roughened surface. For example, the ratio of oxygen to valve metal atoms may be 50% larger than at the protrusions (i.e. in the regions having a higher oxygen to metal atom ratio in the surface layer) than at the non-protruding portions of the rough surface (i.e. in the remainder of the surface layer). Preferably the ratio of oxygen atoms to valve metal atoms is twice as large at the protrusions than at the non-protruding portions of the rough surface.

A suitable method for measuring the ratio is scanning electron microscopy with energy dispersive X-ray spectroscopy, or SEM-EDX.

The bio-effective material incorporated into the treated metal object of the present invention may for instance be a biocidal metal such as copper, ruthenium, silver, gold, platinum or palladium. However, silver is preferable as it is not particularly soluble in body fluids. Other biocidal substances may also be used, for example iodine or chlorhexidine. The adsorbed biocidal material may be any one or any combination of the above.

Where the material incorporated is silver, the silver loading at the surface is 0.1 to 100 µg/cm² of silver. The silver loading is, for example, at least 1 to 20 or 1 to 10 µg/cm²; preferably from 3 to 10 µg/cm² of silver.

The bio-effective material is present in the regions of the surface layer having a higher oxygen to metal atom ratio, at the protrusions upon the micro-rough surface. Typically the bio-effective material is primarily present in the said regions. In detail, the bio-effective material which is adsorbed in the integral surface layer is distributed non-uniformly throughout the surface layer such that some regions have a higher content of bio-effective material than others. The regions having a higher content of bio-effective material than average (that is, the average content of bio-effective material throughout the surface layer) coincide with the regions having a higher oxygen to metal atom ratio. Therefore the regions having a higher content of bio-effective material are located at the protruding portions of the surface. The uneven distribution of bio-effective material throughout the integral surface layer of the metal object of the invention means that the concentration of biological material is greater at the protruding portions of the surface than at the non-protruding portions (for example the troughs).

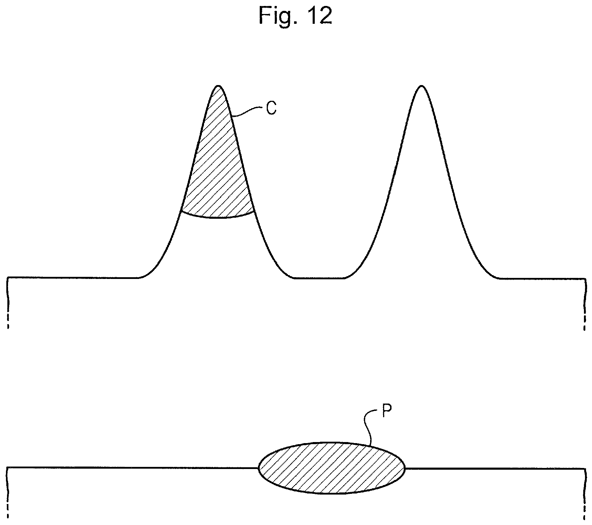

The regions of higher oxygen to metal atom ratio (O/M ratio) and hence the location of the bio-effective material are illustrated in Figure 12. The upper diagram in Figure 12 shows a metal object with a rough surface which has been treated according to the method of the invention. The shaded area, or cap, labelled C, represents a region upon a surface protrusion whereupon a region of high oxygen to metal atom ratio is formed. The lower diagram of Figure 12 shows a metal object with a substantially smooth surface which has been treated according to the method of the invention. The shaded area, or pit, labelled P, represents a region of the surface wherein a pit has formed and which has a higher oxygen to metal atom ratio than the surrounding flat surface.

According to the present invention, the concentration of the bio-effective material located at the surface protrusions (that is, at the regions having a higher oxygen to metal atom ratio) is higher than that in the troughs / valleys. In an embodiment of the invention, the concentration of bio-effective material located at the surface protrusions is at least 20% greater than the concentration of bio-effective material located at the troughs of the surface (that is, the portions of the surface recessed into the object below the mean surface plane). For example, the concentration of bio-effective material located at the surface protrusions is at least 25% greater, preferably at least 30 or 40% greater, more preferably at least 50% greater than the concentration of bio-effective material located at the troughs of the surface. The location of the bio-effective material may be observed, for example, by SEM-EDX.

The treated metal object having a micro-rough surface is typically obtainable, and is preferably obtained, by the method of the invention described above. However, it may also be obtained by methods wherein the anodising voltage is not varied cyclically. An example of such a method is described in published application WO 2009/044203, the entire contents of which is incorporated by reference. For example, a metal object according to the invention may be produced by the following steps:
(a) immersing the metal object having a micro-rough surface in an anodising electrolyte containing a solvent, and passivating the metal to form an anodised integral surface layer on the metal object;
(b) continuing the application of a potential;
(c) producing a hydrous metal oxide by any of (i) applying a negative voltage to the metal object that has been anodised during steps (a and b), while in contact with the anodising electrolyte, or (ii) contacting the metal object that has been anodized with an electrolyte solution containing a reducible soluble salt of titanium or the substrate metal and applying a negative voltage or (iii) contacting the metal object with a chemical reducing agent after steps (a and b); and
(d) removing or separating the anodised metal object resulting from step (c) from the anodising electrolyte, the electrolyte solution or chemical reducing agent, and contacting the anodised metal object with a solution containing a biocidal material so as to incorporate said biocidal material into the surface layer.

### Exemplary method, which is not according to the present invention

The process will now be described in relation to the treatment of a metal implant not comprising microscale protrusions for use in a surgical procedure. The implant is first cleaned, by an aqueous or non-aqueous process (unless it had been sufficiently cleaned during manufacture). The cleaning process may be by ultrasonic cleaning using first acetone (or other degreasing solvent) as the liquid phase, then rinsed with fresh acetone (or other solvent) and then with de-ionized water or any other suitable rinsing solution. The metal implant may then be cleaned in a 1 M aqueous solution of sodium hydroxide (or other alkaline cleaner) and then rinsed in de-ionized water. The resulting cleaned metal implant is then anodised in contact with an aqueous solution of phosphoric acid, which in this example is 2.1 M, as the anodising electrolyte. The implant, in this example, is to be anodised using a maximum voltage of 100 V, to produce a hard wearing anodised oxide layer.

As a preliminary step, the implant is first pre-anodised by linearly increasing the voltage from 0 to 1.75 V, while monitoring the current. This ensures a consistent initial oxide thickness; and from electrical measurements (for example of the film growth current during this pre-anodising step) the microscopic surface area is calculated.

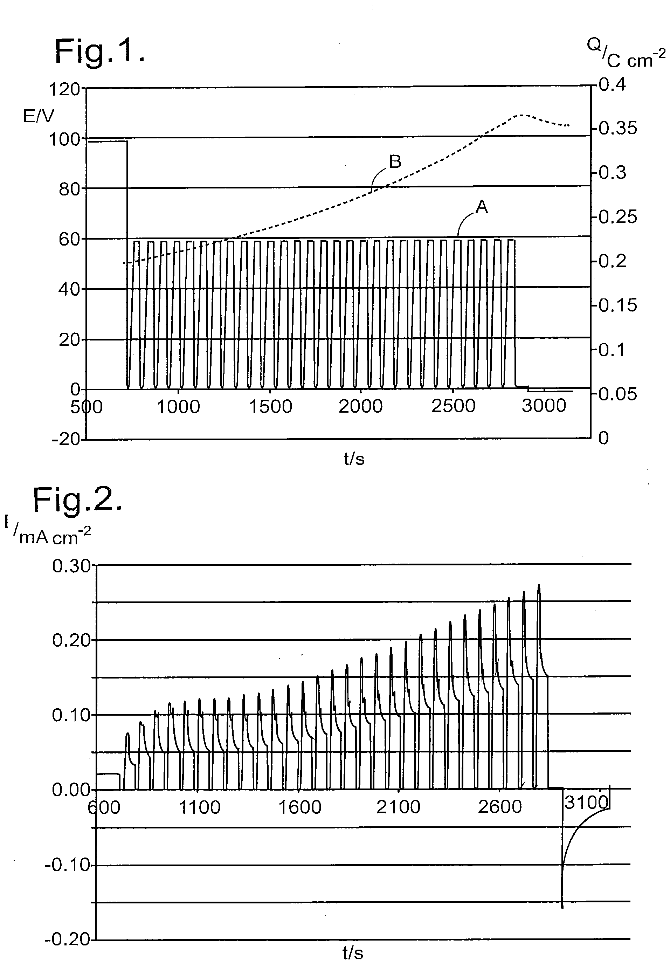

Figures 1 and 2 show experimental observations during treatment of a titanium alloy metal object such as a medical implant; in this case the object is a polished disc of a titanium/aluminium/niobium alloy. The composition of this alloy is more accurately represented as Ti (87%), Nb (7%), Al (6%) in terms of weight, which could be expressed by the empirical chemical formula Ti₂₄NbAl₃. Figure 1 shows a graph, line A, showing the applied voltage, E, used in the anodising process; Figure 2 shows a graph of the corresponding observed current, I, per unit area (with reference to the microscopic surface area); while Figure 1 also shows, line B, the cumulative electric charge, Q, per unit area (with reference to the microscopic surface area).

The electrolyte in this case was 2.1 M aqueous phosphoric acid. During an initial step, the voltage is gradually raised to 100 V, over a period of 100 seconds (at a rate of 1 V/s), and is then held at that voltage for a further 600 s. The anodising process commenced at 0V. The anodising current results in formation of an oxide layer that is integral with the titanium alloy disc, passivating the surface. As the voltage increases, the thickness of the oxide film gradually increases, the relationship being approximately 1.4 nm/V when using this electrolyte. While the voltage is held at that level, the current falls to a low level, for example less than 0.2 mA/cm² (microscopic area) and this drop in current indicates that passivation has been completed. Only the last 220 s of this initial step are shown in figure 1 and only the last 120 s of this initial step are shown in Figure 2.

As shown in figure 1, at a time of 720 seconds, the voltage was dropped to 0, and the titanium alloy disc was then subjected to a sequence of voltage cycles. In each cycle the voltage was swept from 0 to 60 V over a period of 30 seconds (i.e. at 2 V/s), held at 60 V for 30 seconds, then dropped back to 0 over 3 seconds (i.e. at 20 V/s), and held at 0 for 10 seconds. Each cycle therefore lasted 73 seconds. The titanium alloy disc was subjected to 29 such voltage cycles.

As shown in figure 2, the resulting current consists of a corresponding series of current peaks, each of which decreases as the voltage is held at the steady value of 60 V. The peak values of current show a gradual increase during the sequence of voltage cycles. As shown in figure 1, the cumulative charge, as shown by line B, gradually increases throughout the sequence of voltage cycles. The application of voltage cycles is terminated when a desired value of the cumulative charge has been achieved, which in this example was 0.37 C/cm² (microscopic area).

After a brief pause to allow time for insertion of a reference electrode into the electrolyte, and for changing the power supply from a high-voltage supply to a potentiostat (75 s in this case), a reverse voltage of -0.8 V (as measured with respect to a Ag/AgCI standard reference electrode) is applied, and is held for 180 seconds. This negative voltage will bring about electrochemical reduction at the surface. As is evident from figure 2, the resulting reverse current decreases very substantially during that period, and it will be seen that at the end of that period of 180 s the current has dropped to only about 17% of its initial value.

The surface of the titanium alloy disc, at this stage, has a surface layer consisting of a hard oxide layer of a titanium oxide, in which are pits or pitted regions. The hard oxide layer is that formed during the initial passivating step (the first 720 s); the pits are formed during the sequence of voltage cycles (the following 2100 s); and the reverse voltage step (the final 180 s) ensures that the pits are filled with a porous and absorptive material which acts as an effective ion absorber, and which may be a form of hydrous titanium oxide which may contain anions from the electrolyte, such as phosphate, and which was formed by the electrochemical reduction. The porous material in the pits may also contain niobium from the substrate, presumably also as a hydrous oxide.

The pits typically have depths in the range 1 to 3 µm penetrating through the outer passive hard oxide layer (which is 0.14 µm thick at 100 V) into the substrate, and have typical diameters of 1 to 5 µm. The pits may occupy some 5 to 20% of the surface area, though preferably below 10%, so they do not significantly affect the hard wearing properties of the hard oxide layer.

When the anodising steps and the reduction step (as shown in figures 1 and 2) have been completed, the anodised titanium alloy disc is rinsed with de-ionised water to remove phosphoric acid residues and other soluble materials. The thus-cleaned implant is next immersed in a solution comprising the bio-effective material, which is a biocidal material in this example, typically for between 0.5 hours and 2 hours, for example 1 hour. In this example the biocidal material is silver in ionic form, and the solution is an aqueous solution of silver nitrate having a silver concentration in the range of from 0.001 to 10 M, e.g. 0.01 to 1.0 M, for example, 0.1 M or thereabouts, and may have a pH in the range 2 to 7, particularly pH 2.5 to 6.0 or pH 3.3 to 6.0. Silver ions are absorbed within the surface, presumably by ion exchange, the greatest concentration being in the material within the pits. pH values lower than 2 may be used to control silver ion adsorption on very high surface area materials.

The treated titanium alloy disc may have a silver content of 0.5 to 40 µg/cm² or more typically from 2-20 µg/cm² (based on the geometrical surface area). However, titanium or titanium alloy metal objects treated according to the method of the invention may achieve a silver loading of from 0.1 to 100 µg/cm². The silver loading depends upon the anodising process, as described above, and in particular the silver loading is correlated with the cumulative charge, Q, that passes during the anodising steps. The silver is present initially mainly in ionic form but may be at least partially converted to atomic clusters of metal dispersed within the hydrous titania adsorption matrix as a result of photo-reduction. Typically, -0.3-1 µg/cm² (microscopic area) is adsorbed on the hard passive oxide layer, with the remainder stored within the hydrous titania-filled pits.

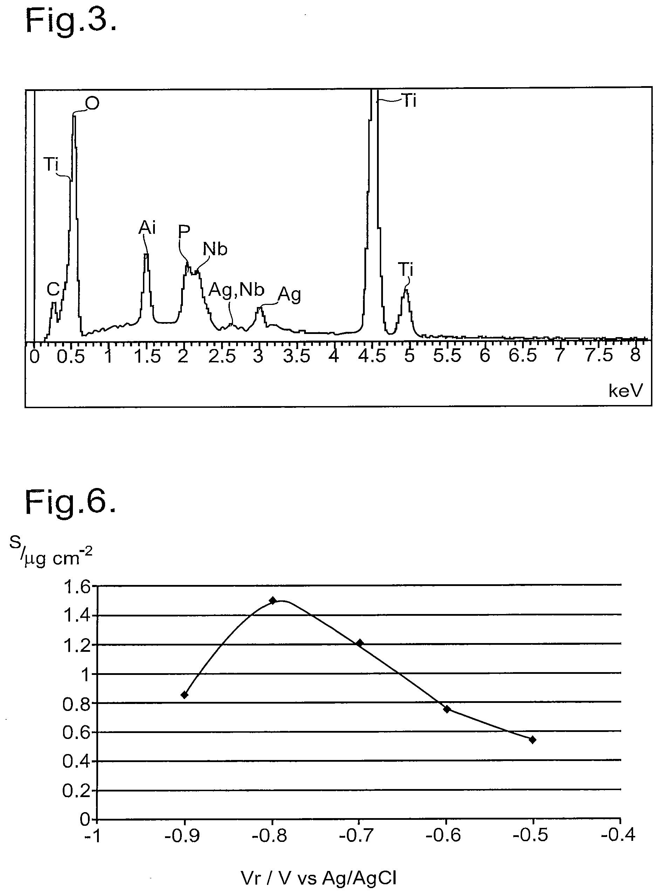

The anodising process typically produces a coloured surface for example as a vivid purple colour or green, because of the thickness of the transparent oxide layer. The vivid purple colour is typically produced at 100 V (or 27 V). Referring now to figure 3, this shows the Energy Dispersive X-Ray (EDX) spectrum from the material in one of the pits, as observed using an electron microscope. The largest peaks in the spectrum are associated with titanium and oxygen, and there are medium-sized peaks associated with titanium, aluminium, phosphorus and niobium, and smaller peaks associated with silver and niobium. It is evident that niobium and titanium from the metal alloy form part of the material within the pits, and also phosphorus and oxygen (from the phosphoric acid electrolyte), and it is evident that silver is also absorbed in the material within the pits.

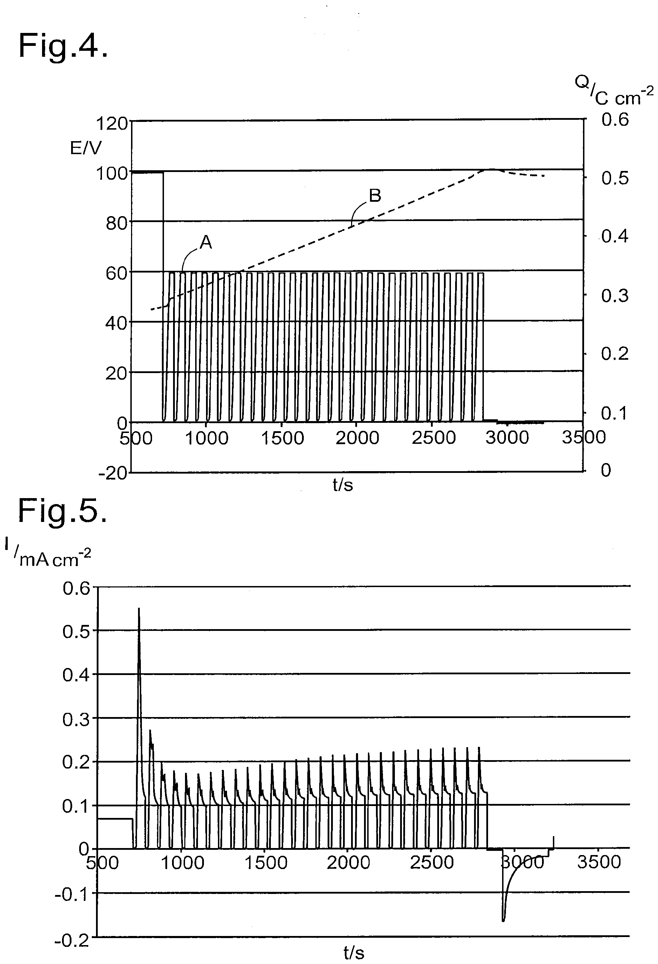

Substantially the same process has also been applied to a titanium alloy disc whose surface had been grit blasted, so the surface was initially rough; this disc was of the same titanium/aluminium/niobium alloy as in the experiments described above with reference to figures 1 and 2. Figure 4 shows a graph, line A, showing the applied voltage, E, used in the anodising process; Figure 5 shows a graph of the corresponding observed current, I, per unit area (with reference to the microscopic surface area); while Figure 4 also shows, line B, the cumulative electric charge, Q, per unit area (with reference to the microscopic surface area). (Note that the scales on the axes for current, I, and cumulative charge, Q, are different from those in figures 1 and 2.)

In this example too the electrolyte is 2.1 M aqueous phosphoric acid. As described above, the disc is first subjected to low-voltage pre-anodisation, so that the microscopic surface area is known. Anodisation is then commenced, and the voltage is gradually raised to 100 V, over a period of 100 seconds (at a rate of 1 V/s), and is then held at that voltage for a further 600 s. The anodisation process commences from 0 V. The anodising current results in formation of an oxide layer that is integral with the titanium alloy disc, passivating the surface. As the voltage increases, the thickness of the oxide film gradually increases, the relationship being approximately 1.4 nm/V with this electrolyte; and while the voltage is held at that level, the current falls to a low level, for example less than 0.1 mA/cm² and this drop in current indicates that passivation has been completed. Only the last 220 s of this initial step are shown in figures 4 and 5.

As shown in figure 4, at a time of 720 seconds, the voltage was dropped to 0, and the titanium alloy disc was then subjected to a sequence of voltage cycles. In each cycle the voltage was swept from 0 to 60 V over a period of 30 seconds (i.e. at 2 V/s), held at 60 V for 30 seconds, then dropped back to 0 over 3 seconds (i.e. at 20 V/s), and held at 0 for 10 seconds. Each cycle therefore lasted 73 seconds. The titanium alloy disc was subjected to 29 such voltage cycles, as with the polished disc described above.

As shown in figure 5, the resulting current consists of a corresponding series of current peaks, each of which decreases as the voltage is held at the steady value of 60 V. The peak values of current start at a high value, rapidly decrease over the first five voltage cycles, and then show a slight increase over the sequence of voltage cycles. As shown in figure 4, the cumulative charge, as shown by line B, gradually increases throughout the sequence of voltage cycles. The application of voltage cycles is terminated when a desired value of the cumulative charge has been achieved, which in this example is 0.51 C/cm².

After a brief pause to allow time for insertion of a reference electrode into the electrolyte, and for changing the power supply from a high-voltage supply to a potentiostat (75 s in this example), a reverse voltage of -0.8 V (as measured with respect to a Ag/AgCI standard reference electrode) is applied, and is held for 180 seconds. This negative voltage will bring about electrochemical reduction at the surface. As is evident from figure 5, the resulting reverse current decreases very substantially during that period, and it will be seen that when the reduction was terminated the current had dropped to only about 10% of its initial value. Typically, the current after 180 seconds is about 4% of its initial value.

The surface of the titanium alloy disc, at this stage, has a surface layer consisting of a hard oxide layer comprising a titanium oxide, in which are pits or pitted regions. The hard oxide layer is that formed during the initial passivating step (the first 720 s); the pits are formed during the sequence of voltage cycles (the following 2100 s); and the reverse voltage step (the final 180 s) ensures that the pits are filled with a porous and absorptive material which acts as an effective ion absorber, and which may be a form of titanium oxide which may also contain some phosphate anions, and which is formed by the electrochemical reduction.

Although the process steps used to treat the grit-blasted titanium alloy disc (as described in relation to figure 4) are the same process steps as used to treat the polished titanium alloy disc (as described in relation to figure 1), the values of current after the first voltage cycle are different, and the variation with time during the voltage cycles is also different.

The grit-blasted titanium alloy disc was then subjected to the rinsing step, and then the silver adsorption step, exactly as described above for the polished titanium alloy disc. Consequently silver ions are absorbed into the surface, in particular within the absorptive material within the pits.

By way of example, the grit-blasted alloy disc was measured as having a geometric area of 2.99 cm², and a microscopic area of 22.93 cm², and so a roughness factor of 7.7. By dissolving silver from the surface into 3 M nitric acid at 30-40°C, with ultrasonic agitation, the total loading of silver was ascertained. The total loading of silver was about 15 µg, so the loading is about 5.0 µg/cm², on a geometric basis.

Tests have also been carried out to ascertain the optimum value of the reverse voltage, applied after anodisation to bring about electrochemical reduction within the pits.

Previous experiments have identified that a reverse voltage of about -0.45 V is optimum if the metal implant is of a titanium/vanadium/aluminium alloy Ti6Al4V (also known as having the empirical chemical formula Ti₂₄VAl₃). These tests investigated a range of different reverse voltages for the titanium/niobium/aluminium alloy described above. A number of polished discs of this titanium metal alloy were subjected to the process described above in relation to figures 1 and 2, except that they were subjected to a range of different values of the reverse voltage, ranging between -0.5 and -0.9 V against a standard Ag/AgCI electrode in each case. In each case the reverse voltage was applied for 180 seconds. In each case also the current rose to an initial peak, and then rapidly decreased, in substantially the same way as shown in figure 2. Furthermore, in each case the larger the reverse voltage, the larger was the current.

As described above, after the anodisation and reverse voltage steps, each disc was rinsed and then immersed in silver nitrate solution, as described above. The loadings of silver ions were then ascertained by dissolving the silver into nitric acid (as described above in relation to the grit-blasted disc).

Referring now to figure 6, this shows how the loading of silver ions, S, measured in µg/cm², varies with the magnitude of the reverse voltage, Vr. The values for the reverse voltage Vr are the observed values of the potential of the disc relative to the standard Ag/AgCI reference electrode. It is clear that the optimum reverse voltage, to obtain the greatest loading of silver, is at between -0.7 and -0.9 V, and is approximately -0.8 V. Other experimental measurements have led to the additional conclusion that the optimum silver loading is achieved if the reverse voltage is initially applied at its peak value, rather than being gradually increased.

So these tests and experiments lead to the conclusion that for this titanium/niobium alloy the potential of the object during the reverse voltage step, relative to the reference electrode, should be about -0.8 V, and that it should be applied at that value initially, rather than building up to that value.

This is a significantly larger reverse voltage than is required with the titanium/vanadium alloy. The difference in behaviour is presumably due to the presence of niobium in the surface oxide (as is evident from the spectrum of Figure 3). This may arise from an effect on the energy bands for electrons within the metal alloy at its interface with the oxide layer.

The anodising method described above has been described primarily in relation to a titanium/niobium alloy, but it will be appreciated that such an anodising method may also be applied to other titanium alloys, or pure titanium, and indeed may be applied to other valve metal alloys. For example if such an anodising process - the passivating step and then the pit-forming step - are applied to a titanium/vanadium alloy, then in the following step the reverse voltage may be lower than is appropriate for the titanium/niobium alloy. It will also be appreciated that the voltage cycles may differ from those described above, for example the voltage might be ramped up and ramped down at different rates, for example being ramped up more slowly (providing a more rapid process). The anodising method has been described in relation to titanium alloy metal discs, but can be applied to any type of item, such as implants, of these suitable metals.

### Exemplary method of the invention applied to an object having a micro-rough surface

The method of the invention will now be described in relation to a method of producing a treated metal object having a micro-rough surface or a smooth surface. Titanium discs are used by way of illustration.

Discs of chemically pure titanium (CpTi) were degreased in acetone, pickled in a mixture of 2% HF and 10% HNO₃ and rinsed in pure water in order to prepare the surface for modification.

The discs were then grit blasted using large grits of corundum with a grain size of 250 to 500 µm. They were then etched in a boiling mixture of hydrochloric and sulphuric acids, and then cleaned in nitric acid, rinsed in ultrapure deionised water in an ultrasonic bath and then dried in a stream of nitrogen. The discs thus produced are referred to hereafter as GBA discs.

Some of these discs were further conditioned for several months in sodium chloride solution. The discs thus produced are referred to hereafter as GBA conditioned discs. Typical conditions used in this step are, for example, a 0.9% solution of sodium chloride, either in water or at a slightly acidic pH (pH 4-6).

Details of the process by which the GBA and GBA conditioned discs as used in the present examples were prepared using the methodology described in given in "Spontaneously formed nanostructures on titanium surfaces", Wennerberg et al., Clin. Oral Impl. Res., 24, 203-209, 2013.

This paper also provides details of the micromorphology of the micro-rough surfaces of the GBA and GBA conditioned discs, and compares them to polished titanium discs. In particular, it illustrates that the micro-rough surfaces comprise features are more akin to peaks than to valleys in the surface. Furthermore, it also illustrates the surface area of the three different types of disc. The surface area of a polished titanium disc is shown to be almost identical to that of a perfectly flat plane having the same geometric shape as the disc. By contrast, the acid-etched discs both have surface areas that are more than twice as large as the area of a plane having the same geometric shape as the discs. The large surface area arises from the micro-rough structure of the acid-etched discs.

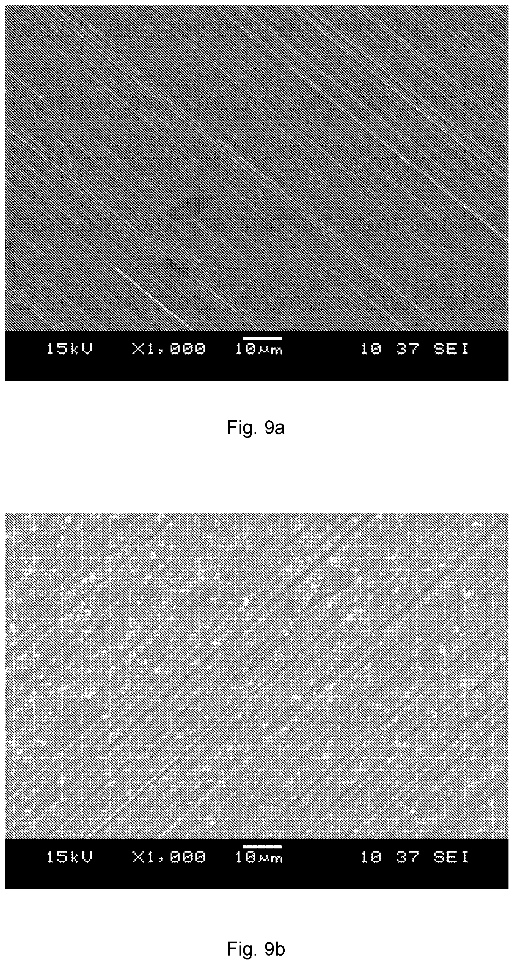

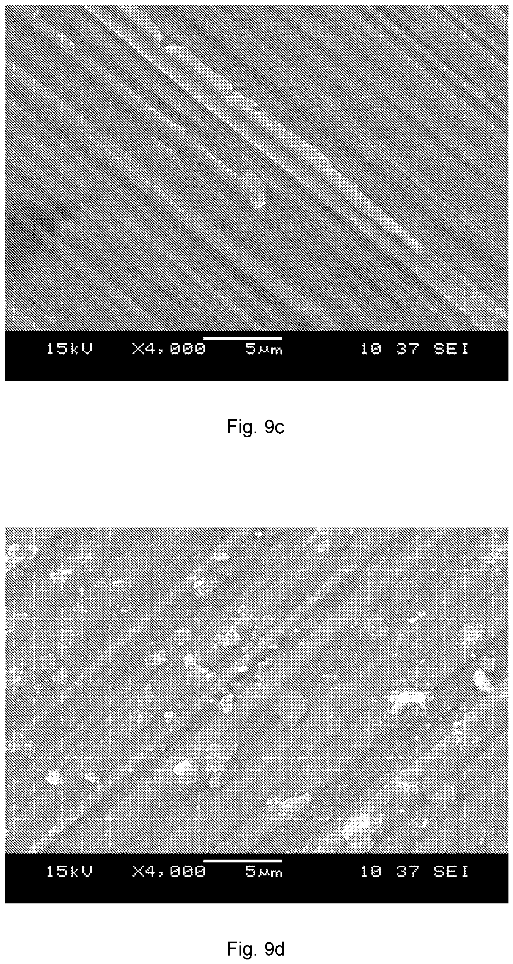

The differences between the three surfaces prior to treatment are illustrated in Figures 7, 8 and 9. Figures 7a, 7c and 7e illustrate the GBA conditioned etched surface, while Figures 8a, 8c and 8e illustrate the GBA surface. Both show sharp microscale features which appear to be peaks and ridges. The GBA conditioned surface, viewed at 15000x magnification in Figure 8e, also appears to have some nanostructure upon the microscale features. By contrast, Figures 9a and 9c show a machine-finished CpTi surface. This surface is essentially flat on the microscale.

All three discs were subjected to a pre-anodisation process as described in WO 2012/095672 A2, the entire contents of which is incorporated herein by reference. The process involves contacting the metal object with a 2M phosphoric acid electrolyte at 20°C and applying a voltage by means of a potentiostat of 0V (compared to a reference electrode). After this, the voltage was linearly increased up to a maximum of 1.75 V, at a rate of 20 mV s⁻¹. The current was recorded at 1.6 V and this value was used to determine the microscopic surface area of the object based on a relationship between oxide film growth current and area. The voltage was then held at the 1.75 V for 2 minutes to ensure that the complete surface was fully passivated.

The anodising procedure was then carried out. The titanium discs were connected to a power supply and the voltage was ramped up from 0V to a maximum of 100 V at a ramp rate of 0.5 V s⁻¹. The voltage was then held at the maximum voltage for 10 minutes in order to produce an integral surface oxide layer. The voltage was then ramped back down to 0 V at a ramp rate of 10 V s⁻¹.

The anodised titanium discs were then subjected to series of voltage cycles wherein the voltage was held at 0 V for 30 s, then ramped up to 36 V at a ramp rate of 2 V s⁻¹ and held at 36 V for 30 s, then ramped back down to 0 V at a ramp rate of 10 V s⁻¹. This was repeated for around 1 to 2 hours, depending on the disc; the exact timings are shown in Table 3 below.

The metal object was then connected to a potentiostat and a voltage of -0.45 V (compared to a reference electrode) was applied for 3 minutes. The metal object was then removed from the phosphoric acid electrolyte and rinsed with type 2 deionised water until the rinse water conductivity was less than 10 µS cm⁻¹.

The metal object was then contacted with a 0.1 M solution of silver nitrate at 18-22°C and a pH of around 3.5 to 3.9 for one hour. The metal object was then removed from the silver nitrate solution and rinsed with type 2 deionised water until the rinse water conductivity was less than 2.5 µS cm⁻¹.

The results are summarised in Table 3 below.

**Table 3. The results observed after treating both machine-finished and rough titanium discs according to the method of the invention. Two measurements of the silver dose were taken on each disc.**

| Parameter | GBA | GBA conditioned | Machined Finish |
|---|---|---|---|
| Electrochemical data | Reduction current observed. | Reduction current observed. | Reduction current observed. |
| Surface Roughness Factor (determined by pre-anodisation technique) | 3.86 | 3.90 | 1.39 |
| SEM | Sharp edges of the surface have been rounded. No clear evidence of | Sharp edges of the surface have been rounded. No clear evidence of | 1-5µm diameter pits observed (match those previously shown to contain |
| | pit formation. | pit formation. | hydrous titania & silver ions). |
| EDX | Silver located on surface - primarily peaks | Silver located on surface - primarily peaks | Silver located on surface primarily in pits. |
| Silver dose (µg/cm²) | 7.15 | 4.88 | 4.91 |
| | 4.10 | 5.10 | 4.73 |

The information in Table 3 summarises the effects of subjecting different types of surface to the process of the invention. An important difference is the location of the adsorbed silver. When the process is applied to a non-roughened surface, it causes the formation of pits approximately 1 to 5 µm in diameter. These are filled with hydrous titania and then adsorb the majority of the silver which is incorporated onto the surface. By contrast, when the process is applied to a microscale roughened (here acid etched) surface, these pits do not form. Instead the surface layer forms with particularly high oxygen atom content at the sharp edges of the surface microscale features, giving them a rounded appearance. The subsequent immersion in silver nitrate solution results in the adsorption of silver particularly on the protruding features (as measured by SEM-EDX).

The differences in surface structure between the treated titanium discs are illustrated in Figures 7, 8 and 9. Figure 7 shows the GBA conditioned disc, in both treated and untreated form, at various different magnifications. Comparing the images of the treated and untreated discs shows that the integral surface layer produced by the anodisation process and subsequent steps has two clear effects. It rounds the sharp edges of the structure, and engulfs any nanostructure at the surface. However, no pits are visible in the surface; the integral surface layer structure appears to follow closely that of the metal object underneath. Very similar results are seen in the case of the GBA disc: the sharp edges of the surface are rounded by the formation of the integral surface layer.

By contrast, the structure produced by treatment of the smoother machine-finished disc does not mimic the structure of the underlying flat surface. Instead, the process produces localised pits which contain the majority of the adsorbed silver.

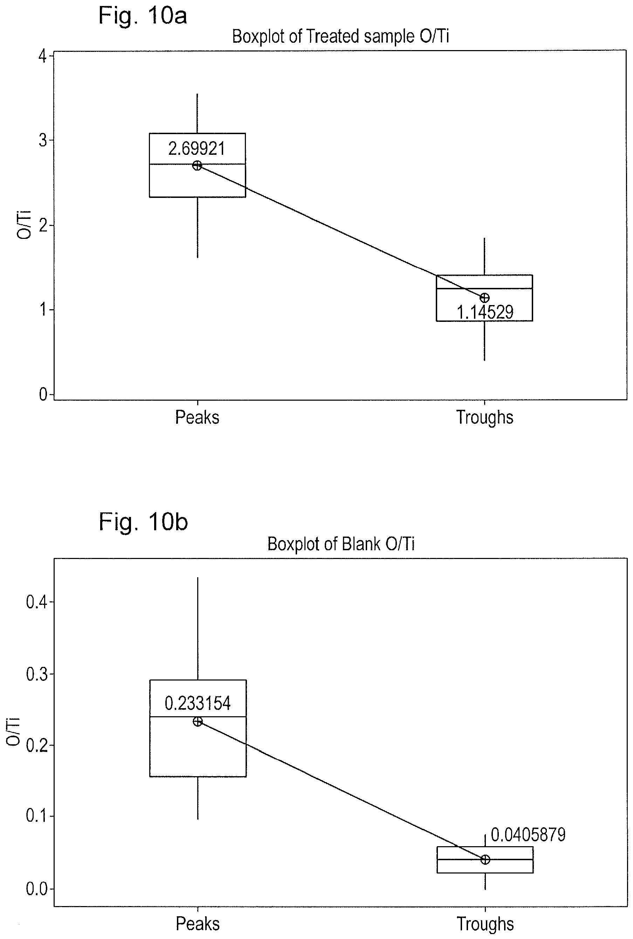

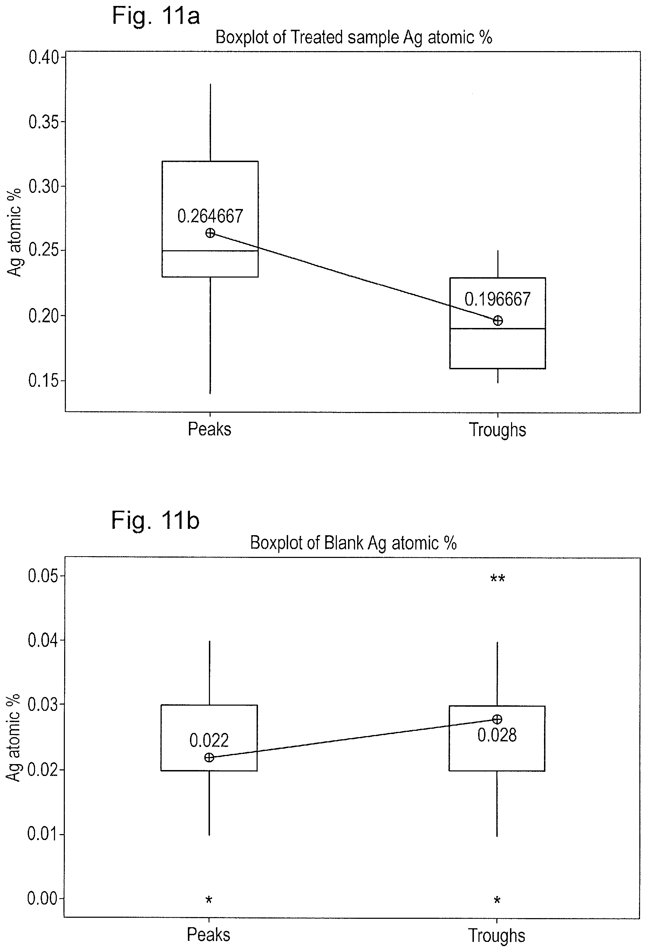

As discussed above, SEM-EDX analysis can be used to illustrate the uneven atomic composition of the surface layer across the rough surface of the metal object of the invention. SEM-EDX analysis was performed to obtain the results shown in Figures 10 and 11. A JEOL 6480 LV SEM equipped with an Oxford Instruments X-MAX80 SD X-ray detector and INCA X-ray analysis system was used to image the samples and perform the analysis using EDX. EDX analyses the characteristic X-rays produced by the interaction between the primary electron beam and the sample. The technique identifies all elements present with atomic numbers of 5 and greater (boron) with a detection limit of approximately 0.1 weight %. The experiment was run using an accelerating voltage of 15kV, a chamber pressure of 30 Pascal (air), and a probe current of 1 nano-amp. At a magnification of x15,000, the operator picked 3 peaks & 3 troughs/valleys in each of five areas distributed across the whole of the disc and collected EDX data (2 minutes count time) for Ti, O & Ag for two different discs. These were a GBA conditioned disc treated according to the method of the invention, and a blank GBA conditioned disc, treated only according to the last step of the method of the invention (exposure to a solution of bio-effective material, specifically silver).

Figure 10c illustrates what is meant by a peak and a trough, in terms of the surface profile of the discs. A peak is an area of the disc which protrudes above the mean surface plane, away from the metal object, while a trough is a region recessed into the disc below the mean surface plane.

Figure 10a shows the O/Ti ratio of a GBA conditioned disc treated according to the method of the invention. It shows that there is a significant difference (p = 0.000) between the O/Ti ratio at the peaks, which is approximately 2.7 and is ascribed to dehydrated hydrous titania, and the O/Ti ratio at the troughs, which is approximately 1.1 and is ascribed to TiO. Figure 10b shows that there is a significant difference (p = 0.000) between these regions on the blank disc as well. The mean O/Ti ratio for a peak is approximately 0.2, and there is also a positive skew to the data. The mean O/Ti ratio for the troughs is effectively 0. These figures illustrate that the method of the invention creates a surface layer containing at least one oxide of the metal, and that said layer contains regions of higher and lower oxygen to metal atom ratios, with the higher-ratio regions being located at the peaks upon the surface.

Figure 11a shows the silver content in atomic % of a GBA conditioned disc treated according to the method of the invention. It shows that there is a significant difference (p = 0.002) between the silver content of a peak compared to a trough on the treated sample, with the amount of silver present upon the peaks being higher than the amount of silver in the troughs. Thus Figure 11a shows that bio-effective material is primarily adsorbed at the regions of higher oxygen to metal atom ratio of the surface layer.

Figure 11b shows the silver content in atomic % of a GBA conditioned disc treated only according to the last step of the method of the invention, that is exposure to a solution of bio-effective material, in this case silver. By contrast to Figure 11a, the blank disc of Figure 11b shows that there is no significant difference (p = 0.175) between the silver content of peaks and troughs in the blank sample. The outliers at the upper end indicate positive skewness, which is logical because at the lower end of the distribution, no values can be less than zero.

This data illustrates that the oxygen content of the roughened surfaces differ at the protruding and non-protruding portions of the rough surfaces. More oxygen is located at the protruding portions. On all rough surfaces examined, the O/Ti ratio is greater in the peaks than in the valleys. This is a pronounced difference, not a minor one; on the roughened surfaces, the O/Ti ratio is at least twice as great at the peaks than in the valleys. By contrast, anodisation of a metal object having a machine-finished surface leads to the presence of a larger O/Ti ratio at pits in the surface, rather than at surface protrusions. Moreover, in the case of metal objects having machine-finished surfaces, the regions of high oxygen content (the pits) have an O/Ti ratio which is larger than but not twice as large as the regions of lower oxygen content.

The data in Figure 10 also show that the O/Ti ratio is greater than 1.5 at the peaks on all treated roughened surfaces examined. This contrasts the O/Ti ratio at the valleys between the protrusions from such surfaces which have a much lower O/Ti ratio.

Very surprisingly, despite the vastly different surface structure, the silver content achieved at the surface appears to be remarkably similar on micro-rough surfaces (where the silver is primarily present at the caps) and on smooth surfaces (where the silver is largely present in pits). The silver loading values summarised in Table 3 above are remarkably similar, all being in the region of 5 µg cm⁻². Such a dosage is believed to be biologically effective, as is discussed below, but sufficiently low as to be non-toxic. It is most surprising that similar dosages are achieved, regardless of the differing surface area of the surfaces tested and the differing structures into which the silver is adsorbed.

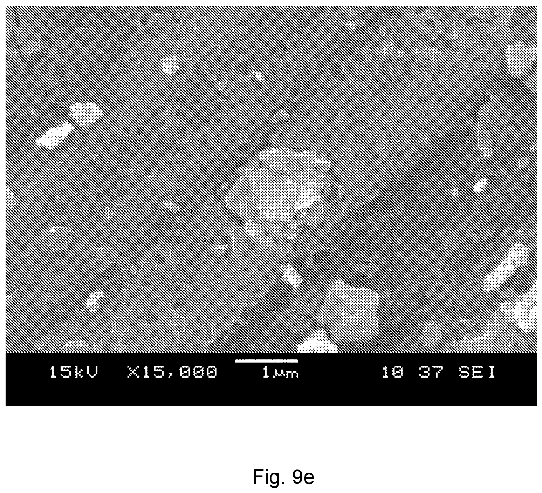

For the purposes of comparison, the method as described above was applied to a machine finished titanium surface. The outcome can be seen in Figures 9b, 9d and 9e, which clearly show the formation of pits on the surface. An SEM-EDX analysis was performed on the treated machine-finished surface (data not shown) and it was found that the O/Ti ratio was significantly higher at the pits than elsewhere on the machine-finished surface. The O/Ti ratio at the pits was typically between 2 and 3, whereas the O/Ti ratio elsewhere upon the surface was typically less than 2, usually between 1 and 1.6. This finding contrasts the findings in relation to micro-rough surfaces, which display a higher O/Ti ratio at the protruding portions of the surface than at the troughs or valleys.

Incorporation of silver appears to be achievable twice as quickly at acid-etched surfaces than on machine-finished surfaces, which is a further advantage of using a roughened surface in the method of the invention, in addition to the advantage of being suitable for osseointegration and tissue attachment.

In use of the treated metal object it is thought that during exposure to body fluids there is a slow leaching of silver species from the anodised layer, so that the growth of microorganisms such as bacteria, yeasts or fungi in the vicinity of the metal object is inhibited. The leaching is thought to be effected by ion exchange of silver on the metal object with sodium in the body fluids that contact the metal object.

It is to be understood that references herein to silver as a biocidal metal also apply to other biocidal metals, such as copper, ruthenium, gold, platinum, palladium or mixtures thereof, either alone or in combination with other biocidal metal(s). Other biocidal substances may also be used, for example iodine or chlorhexidine.

## Claims

1. A metal object having a micro-rough surface and an anodised surface layer thereon which is integral with the metal object, wherein:
the micro-rough surface of the metal object comprises microscale protrusions;
the integral surface layer comprises at least one oxide of the metal and the oxygen in the surface layer is non-uniformly distributed across the surface of the metal object such that there are regions having a higher O/M ratio than the average O/M ratio in the anodised surface layer, where O is the number of oxygen atoms and M is the number of valve metal atoms, the regions being located on said microscale protrusions; and
a bio-effective material is incorporated into the regions of higher O/M ratio.

2. The metal object of claim 1 wherein the bio-effective material is a biocidal material, preferably silver.

3. The metal object of claim 1 or claim 2 wherein the metal object comprises titanium or alloys thereof.

4. The metal object of any one of claims 1 to 3 wherein the metal object is an implant, optionally an orthopaedic implant or a cardiac implant.

5. The metal object of any one of claims 1 to 4 wherein the rough surface of the metal object has an average roughness (R_{A}) of up to 3 µm; and/or wherein the rough surface of the metal object has an average roughness (R_{A}) of from 0.5 to 5 µm and a peak density of at least 1000 mm⁻².

6. The metal object of any one of claims 1 to 5 wherein the rough surface is an acid etched surface.

7. The metal object of any one of claims 1 to 6 wherein the concentration of bio-effective material located at the surface protrusions, having a higher oxygen to valve metal atom ratio is at least 20% greater than the concentration of bio-effective material located at the troughs of the surface.

8. The metal object of any one of claims 1 to 7 wherein the ratio of oxygen atoms to valve metal atoms in the integral surface layer is at least two at the microscale protrusions, and between one and two at the non-protruding portions of the surface.

9. A method of treating a metal object so as to form thereon a surface layer which is integral with the metal object, and which includes a bio-effective material, the method comprising the following steps:
(a) contacting a metal object having a micro-rough surface comprising microscale protrusions with an anodising electrolyte, and applying an anodising voltage to the metal object to passivate the metal by forming an anodised oxide layer on the metal object;
(b) continuing the application of an anodising voltage to produce regions in the oxide layer having a higher O/M ratio, where O is the number of oxygen atoms and M is the number of valve metal atoms;
(c) producing a hydrous metal oxide in said regions in the oxide layer by electrochemical or chemical reduction in contact with an electrolyte or a solution, so the oxide layer and the hydrous metal oxide in said regions together constitute the surface layer;
(d) removing or separating the anodised metal object resulting from step (c) from the electrolyte or the solution of step (c); and
(e) contacting the anodised metal object with a solution containing a bio-effective material so as to incorporate said bio-effective material into the surface layer, the bio-effective material preferably being a biocidal material, preferably silver;
*wherein* during step (b) the anodising voltage is repeatedly subjected to voltage cycles, each voltage cycle comprising ramping the voltage between a lower threshold voltage and an upper threshold voltage, and then returning the voltage to the lower threshold voltage, both the lower threshold voltage and the upper threshold voltage being less than the maximum voltage applied during the passivating step (a).

10. A method as claimed in claim 9 wherein the method comprises a surface roughening step prior to step (a) in order to produce the metal object having a micro-rough surface comprising microscale protrusions, wherein the method of roughening optionally comprises acid etching and wherein after the acid-etching step and prior to step (a), the metal object is optionally conditioned in a solution of sodium chloride.

11. A method as claimed in claim 9 or 10 wherein the lower threshold voltage is below 25 V, while the upper threshold voltage is above 35 V, preferably the upper threshold voltage is between 35 V and 70 V, preferably between 40 V and 70 V, and the lower threshold voltage is 0 V.

12. A method as claimed in any one of claims 9 to 11
(i) wherein, while voltage is ramped, it is varied continuously, optionally at a rate between 0.5 and 15 V/s, preferably between 0.5 and 5 V/s; and/or
(ii) wherein, while the voltage is returned to the lower threshold voltage, it is decreased more rapidly than it is increased while being ramped to the upper threshold voltage; and/or
(iii) wherein the method comprises monitoring the total electrical charge that has passed during the application of the anodising voltages in steps (a) and (b), and ensuring that the total electrical charge per unit area lies within a predetermined desired range; and/or
(iii) wherein electrochemical reduction is performed in step (c), and the magnitude of the negative voltage during electrochemical reduction is maintained or regulated so as to be insufficient to cause electrolysis of the solvent.

13. A method as claimed in any one of claims 9, 11 or 12 wherein electrochemical reduction is performed in step (c), with application of a reverse voltage, wherein the object comprises a titanium/niobium alloy, and the reverse voltage is applied such that the potential of the object is between -0.7 V and -0.9 V relative to a standard Ag/AgCI electrode.

14. A method as claimed in claim 10 wherein the metal object comprises titanium; in step (b) the upper threshold voltage is between 30 V and 70 V and the lower threshold voltage is 0 V; while the voltage is ramped it is varied at a rate of between 0.5 and 15 V/s; and electrochemical reduction is performed in step (c), wherein a reverse voltage is applied such that the potential of the object is between -0.1 and -0.9 V relative to a standard Ag/AgCI electrode.

15. The metal object of any one of claims 1 to 8, wherein the metal object is obtainable by the method as claimed in any one of claims 9 to 14.

## Patentansprüche

1. Metallobjekt mit einer mikrorauen Oberfläche und einer darauf anodisierten Oberflächenschicht, die einstückig mit dem Metallobjekt ist, wobei:
die mikrorauhe Oberfläche des Metallobjekts mikroskalige Vorsprünge umfasst;
die einstückige Oberflächenschicht mindestens ein Oxid des Metalls umfasst und der Sauerstoff in der Oberflächenschicht ungleichmäßig über die Oberfläche des Metallobjekts verteilt ist, so dass Bereiche vorhanden sind, die ein höheres O/M-Verhältnis als das durchschnittliche O/M-Verhältnis in der anodisierten Oberflächenschicht aufweisen, wobei O die Anzahl der Sauerstoffatome und M die Anzahl der Ventilmetallatome ist, wobei sich die Bereiche auf den mikroskaligen Vorsprüngen befinden; und
ein bioeffektives Material in die Bereiche mit höherem O/M-Verhältnis eingearbeitet wird.

2. Metallobjekt nach Anspruch 1, wobei das bioeffektive Material ein biozides Material ist, vorzugsweise Silber.

3. Metallobjekt nach Anspruch 1 oder Anspruch 2, wobei das Metallobjekt Titan oder Legierungen davon umfasst.

4. Metallobjekt nach einem der Ansprüche 1 bis 3, wobei das Metallobjekt ein Implantat, optional ein orthopädisches Implantat oder ein Herzimplantat ist.

5. Metallobjekt nach einem der Ansprüche 1 bis 4, wobei die raue Oberfläche des Metallobjekts eine durchschnittliche Rauheit (R_{A}) von bis zu 3 µm aufweist; und/oder wobei die raue Oberfläche des Metallobjekts eine durchschnittliche Rauheit (R_{A}) von 0,5 bis 5 µm und eine Spitzendichte von mindestens 1000 mm⁻² aufweist.

6. Metallobjekt nach einem der Ansprüche 1 bis 5, wobei die raue Oberfläche eine säuregeätzte Oberfläche ist.

7. Metallobjekt nach einem der Ansprüche 1 bis 6, wobei die Konzentration an bioeffektivem Material, das sich an den Oberflächenvorsprüngen befindet und ein höheres Verhältnis von Sauerstoff zu Ventilmetallatomen aufweist, mindestens 20% höher ist als die Konzentration an bioeffektivem Material, das sich an den Mulden der Oberfläche befindet.

8. Metallobjekt nach einem der Ansprüche 1 bis 7, wobei das Verhältnis von Sauerstoffatomen zu Ventilmetallatomen in der einstückigen Oberflächenschicht an den mikroskaligen Vorsprüngen mindestens zwei und an den nicht hervorstehenden Abschnitten der Oberfläche zwischen eins und zwei beträgt.

9. Verfahren zum Behandeln eines Metallobjekts, um darauf eine Oberflächenschicht zu bilden, die einstückig mit dem Metallobjekt ist und ein bioeffektives Material umfasst, wobei das Verfahren die folgenden Schritte umfasst:
(a) Inkontaktbringen eines Metallobjekts, das eine mikroraue Oberfläche aufweist, die mikroskalige Vorsprünge umfasst, mit einem anodisierenden Elektrolyten, und Anlegen einer Anodisierungsspannung an das Metallobjekt, um das Metall durch Bildung einer anodisierten Oxidschicht auf dem Metallobjekt zu passivieren;
(b) Fortsetzen des Anlegens einer Anodisierungsspannung zur Erzeugung von Bereichen in der Oxidschicht mit einem höheren O/M-Verhältnis, wobei O die Anzahl der Sauerstoffatome und M die Anzahl der Ventilmetallatome ist;
(c) Erzeugen eines wasserhaltigen Metalloxids in den Bereichen in der Oxidschicht durch elektrochemische oder chemische Reduktion in Kontakt mit einem Elektrolyten oder einer Lösung, so dass die Oxidschicht und das wasserhaltige Metalloxid in den Bereichen zusammen die Oberflächenschicht bilden;
(d) Entfernen oder Trennen des anodisierten Metallobjektes, das aus Schritt (c) resultiert, aus dem Elektrolyten oder der Lösung von Schritt (c); und
(e) Inkontaktbringen des anodisierten Metallobjekts mit einer Lösung, die ein bioeffektives Material enthält, um das bioeffektive Material in die Oberflächenschicht zu integrieren, wobei das bioeffektive Material vorzugsweise ein biozides Material ist, vorzugsweise Silber;
*wobei* während Schritt (b) die Anodisierungsspannung wiederholt Spannungszyklen ausgesetzt wird, wobei jeder Spannungszyklus das Erhöhen der Spannung zwischen einer unteren Schwellenspannung und einer oberen Schwellenspannung und das anschließende Zurückführen der Spannung zur unteren Schwellenspannung umfasst, wobei sowohl die untere Schwellenspannung als auch die obere Schwellenspannung kleiner sind als die maximale Spannung, die während des Passivierungsschritts (a) angelegt wird.

10. Verfahren nach Anspruch 9, wobei das Verfahren einen Oberflächenaufrauungsschritt vor Schritt (a) umfasst, um das Metallobjekt mit einer mikrorauen Oberfläche mit mikroskaligen Vorsprüngen herzustellen, wobei das Aufrauungsverfahren optional Säureätzen umfasst und nach dem Säureätzschritt und vor Schritt (a) das Metallobjekt optional in einer Natriumchloridlösung konditioniert wird.

11. Verfahren nach Anspruch 9 oder 10, wobei die untere Schwellenspannung unter 25 V liegt, während die obere Schwellenspannung über 35 V liegt, wobei die obere Schwellenspannung vorzugsweise zwischen 35 V und 70 V liegt, vorzugsweise zwischen 40 V und 70 V liegt und die untere Schwellenspannung 0 V beträgt.

12. Verfahren nach einem der Ansprüche 9 bis 11,
(i) wobei die Spannung, während sie erhöht wird, kontinuierlich variiert wird, optional mit einer Geschwindigkeit zwischen 0,5 und 15 V/s, vorzugsweise zwischen 0,5 und 5 V/s; und/oder
(ii) wobei die Spannung, während sie auf die untere Schwellenspannung zurückgeführt wird, schneller verringert wird als sie erhöht wird, während sie auf die obere Schwellenspannung hochgefahren wird; und/oder
(iii) wobei das Verfahren Folgendes umfasst: Überwachen der gesamten elektrischen Ladung, die während des Anlegens der Anodisierungsspannungen in den Schritten (a) und (b) geflossen ist, und Sicherstellen, dass die gesamte elektrische Ladung pro Flächeneinheit innerhalb eines vorbestimmten gewünschten Bereichs liegt; und/oder
(iii) wobei die elektrochemische Reduktion in Schritt (c) durchgeführt wird, und die Größe der negativen Spannung während der elektrochemischen Reduktion beibehalten oder reguliert wird, so dass sie nicht ausreicht, um eine Elektrolyse des Lösungsmittels zu bewirken.

13. Verfahren nach einem der Ansprüche 9, 11 oder 12, wobei die elektrochemische Reduktion in Schritt (c) unter Anlegen einer Sperrspannung durchgeführt wird, wobei das Objekt eine Titan/Niob-Legierung umfasst und die Sperrspannung so angelegt wird, dass das Potential des Objekts zwischen -0,7 V und -0,9 V relativ zu einer Standard-Ag/AgCl-Elektrode liegt.

14. Verfahren nach Anspruch 10, wobei das Metallobjekt Titan umfasst; in Schritt (b) die obere Schwellenspannung zwischen 30 V und 70 V liegt und die untere Schwellenspannung 0 V beträgt; während die Spannung hochgefahren wird, sie mit einer Geschwindigkeit zwischen 0,5 und 15 V/s variiert wird; und die elektrochemische Reduktion in Schritt (c) durchgeführt wird, wobei eine Sperrspannung angelegt wird, so dass das Potential des Objekts zwischen -0,1 und -0,9 V relativ zu einer Standard-Ag/AgCl-Elektrode liegt.

15. Metallobjekt nach einem der Ansprüche 1 bis 8, wobei das Metallobjekt durch das Verfahren nach einem der Ansprüche 9 bis 14 erhältlich ist.

## Revendications

1. Objet en métal ayant une surface micro-rugueuse et une couche de surface anodisée sur celle-ci qui est monobloc avec l'objet en métal, dans lequel :
la surface micro-rugueuse de l'objet en métal comprend des protubérances à micro-échelle ;
la couche de surface monobloc comprend au moins un oxyde du métal et l'oxygène dans la couche de surface est distribué non uniformément sur la surface de l'objet en métal de telle sorte qu'il y ait des régions ayant un rapport O/M plus élevé que le rapport O/M moyen dans la couche de surface anodisée, où O est le nombre d'atomes d'oxygène et M est le nombre d'atomes de métal valve, les régions étant situées sur lesdites protubérances à micro-échelle ; et
un matériau bio-efficace est incorporé dans les régions de rapport O/M plus élevé.

2. Objet en métal selon la revendication 1, dans lequel le matériau bio-efficace est un matériau biocide, de préférence de l'argent.

3. Objet en métal selon la revendication 1 ou la revendication 2, dans lequel l'objet en métal comprend du titane ou des alliages de ceux-ci.

4. Objet en métal selon l'une quelconque des revendications 1 à 3, dans lequel l'objet en métal est un implant, optionnellement un implant orthopédique ou un implant cardiaque.

5. Objet en métal selon l'une quelconque des revendications 1 à 4, dans lequel la surface rugueuse de l'objet en métal a une rugosité moyenne (R_{A}) d'au plus 3 µm ; et/ou dans lequel la surface rugueuse de l'objet en métal a une rugosité moyenne (R_{A}) de 0,5 à 5 µm et une densité maximale d'au moins 1000 mm⁻².

6. Objet en métal selon l'une quelconque des revendications 1 à 5 dans lequel la surface rugueuse est une surface gravée à l'acide.

7. Objet en métal selon l'une quelconque des revendications 1 à 6 dans lequel la concentration en matériau bio-efficace situé au niveau des protubérances de surface, ayant un rapport d'atomes oxygène-métal valve plus élevé est au moins supérieur de 20 % à la concentration en matériau bio-efficace situé au niveau des creux de la surface.

8. Objet en métal selon l'une quelconque des revendications 1 à 7 dans lequel le rapport d'atomes d'oxygène par rapport aux atomes de métal valve dans la couche de surface monobloc est d'au moins deux au niveau des protubérances à micro-échelle, et entre un et deux au niveau des portions, ne faisant pas saillie, de la surface.

9. Procédé pour traiter un objet en métal afin de former sur celui-ci une couche de surface qui est monobloc avec l'objet en métal, et qui inclut un matériau bio-efficace, le procédé comprenant les étapes suivantes :
(a) la mise en contact d'un objet en métal ayant une surface micro-rugueuse comprenant des protubérances à micro-échelle avec un électrolyte d'anodisation, et l'application d'une tension d'anodisation sur l'objet en métal pour passiver le métal en formant une couche d'oxyde anodisée sur l'objet en métal ;
(b) la continuation de l'application d'une tension d'anodisation pour produire des régions dans la couche d'oxyde ayant un rapport O/M plus élevé, où O est le nombre d'atomes d'oxygène et M est le nombre d'atomes de métal valve ;
(c) la production d'un oxyde de métal hydraté dans lesdites régions dans la couche d'oxyde par réduction électrochimique ou chimique en contact avec un électrolyte ou une solution, pour que la couche d'oxyde et l'oxyde de métal hydraté dans lesdites régions constituent ensemble la couche de surface ;
(d) l'enlèvement ou la séparation de l'objet en métal anodisé résultant de l'étape (c) à partir de l'électrolyte ou de la solution de l'étape (c) ; et
(e) la mise en contact de l'objet en métal anodisé avec une solution contenant un matériau bio-efficace afin d'incorporer ledit matériau bio-efficace dans la couche de surface, le matériau bio-efficace étant de préférence un matériau biocide, de préférence de l'argent ;
dans lequel, durant l'étape (b), la tension d'anodisation est soumise de façon répétée à des cycles de tension, chaque cycle de tension comprenant l'accroissement de la tension entre une tension de seuil inférieure et une tension de seuil supérieure, et puis la remise de la tension à la tension de seuil inférieure, la tension de seuil inférieure et la tension de seuil supérieure étant toutes les deux inférieures à la tension maximum appliquée durant l'étape de passivation (a).

10. Procédé selon la revendication 9, dans lequel le procédé comprend une étape de rugosification de surface avant l'étape (a) afin de produire l'objet en métal ayant une surface micro-rugueuse comprenant des protubérances à micro-échelle, dans lequel le procédé de rugosification comprend optionnellement une gravure à l'acide et dans lequel, après l'étape de gravure à l'acide et avant l'étape (a), l'objet en métal est optionnellement conditionné dans une solution de chlorure de sodium.

11. Procédé selon la revendication 9 ou 10 dans lequel la tension de seuil inférieure est inférieure à 25 V, alors que la tension de seuil supérieure est supérieure à 35 V, de préférence la tension de seuil supérieure est entre 35 V et 70 V, de préférence entre 40 V et 70 V, et la tension de seuil inférieure est de 0 V.

12. Procédé selon l'une quelconque des revendications 9 à 11
(i) dans lequel, lorsque la tension est accrue, elle est variée en continu, optionnellement à un taux entre 0,5 et 15 V/s, de préférence entre 0,5 et 5 V/s ; et/ou
(ii) dans lequel, lorsque la tension est remise à la tension de seuil inférieure, elle est réduite plus rapidement qu'elle n'est augmentée lorsqu'elle est accrue jusqu'à la tension de seuil supérieure ; et/ou
(iii) dans lequel le procédé comprend la surveillance de la charge électrique totale qui est passée durant l'application des tensions d'anodisations dans les étapes (a) et (b), et la garantie que la charge électrique totale par unité de surface se trouve au sein d'une plage souhaitée prédéterminée ; et/ou
(iii) dans lequel une réduction électrochimique est réalisée dans l'étape (c), et l'amplitude de la tension négative durant la réduction électrochimique est maintenue ou régulée afin d'être insuffisante pour causer l'électrolyse du solvant.

13. Procédé selon l'une quelconque des revendications 9, 11 ou 12, dans lequel une réduction électrochimique est réalisée dans l'étape (c), avec l'application d'une tension inverse, dans lequel l'objet comprend un alliage de titane/niobium, et la tension inverse est appliquée de telle sorte que le potentiel de l'objet soit entre -0,7 V et -0,9 V relativement à une électrode d'Ag/AgCl standard.

14. Procédé selon la revendication 10, dans lequel l'objet en métal comprend du titane ; dans l'étape (b) la tension de seuil supérieure est entre 30 V et 70 V et la tension de seuil inférieure est de 0 V ; lorsque la tension est accrue elle est variée à un taux d'entre 0,5 et 15 V/s ; et une réduction électrochimique est réalisée dans l'étape (c), dans lequel une tension inverse est appliquée de telle sorte que le potentiel de l'objet soit entre -0,1 et -0,9 V relativement à une électrode d'Ag/AgCl standard.

15. Objet en métal selon l'une quelconque des revendications 1 à 8, dans lequel l'objet en métal peut être obtenu par le procédé selon l'une quelconque des revendications 9 à 14.
